# EUROPEAN PATENT APPLICATION

(11) **EP 4 592 393 A1**
(43) Date of publication of application: **30.07.2025**
(21) Application number: 23868171.2
(22) Date of filing: 19.09.2023
(51) Int. Cl.: C12N 15/55, C12N 1/15, C12N 1/19, C12N 1/21, C12N 9/20, C12N 15/10, C12N 15/63

(54) **LIPASE VARIANT**

(30) Priority: 20.09.2022 JP 2022149382
(71) Applicant: Kao Corporation, Chuo-ku, Tokyo 103-8210 (JP)
(72) Inventor: HIOKI, Takahiro, Wakayama-shi, Wakayama 640-8580 (JP); KAWAHARA, Akihito, Wakayama-shi, Wakayama 640-8580 (JP)
(74) Representative: Vossius & Partner Patentanwälte Rechtsanwälte mbB
(86) International application number: PCT/JP2023/033910
(87) International publication number: WO 2024/063050

(57) **Abstract**

Provided is a lipase mutant with improved heterologous expression. The lipase mutant consists of an amino acid sequence obtained by substituting an amino acid residue at a predetermined position numbered according to SEQ ID NO: 2 with a predetermined amino acid residue in an amino acid sequence of a parent lipase.

## Description

### Field of the Invention

The present invention relates to a lipase mutant.

### Background of the Invention

Lipases contribute to removal of dirt including oil by hydrolyzing ester bonds in lipids to generate fatty acids and are useful in cleansing applications. As a lipase useful for cleansing, a *Thermomyces lanuginosus-*derived lipase (hereinafter, TLL) is commercially available under the trade name LIPOLASE (registered tradename). Patent Literature 1 discloses that *Cedecea* sp-16640 strain-derived lipase Lipr139 exhibits excellent cleansing performance compared to TLL. Patent Literature 2 discloses a mutant of a Proteus bacterium-derived lipase (hereinafter, PvLip), having improved cleansing performance compared to one or more reference lipolytic enzymes. Patent Literature 3 discloses that a metagenome-derived lipase Lipr138 exhibits excellent cleansing performance compared to TLL. Lipr139, PvLip, and Lipr138 are lipases belonging to the same clade (*Proteus*/*Yersinia* clade) that includes lipases derived from a *Proteus* bacterium or *Yersinia* bacterium.

Many lipases derived from gram-negative bacteria require a lipase-specific chaperone for folding into the active form (Non Patent Literature 1) and have a challenge in low-cost manufacturing by heterologous expression. It has been reported that the heterologous expression is improved by coexpression with a specific chaperone (Non Patent Literature 2), but the productivity thereof is low, and there is still a large challenge in low-cost manufacturing by heterologous expression. In contrast, the bacterial lipases of the *Proteus*/*Yersinia* clade do not require a specific chaperone and have an advantage in low-cost manufacturing by heterologous expression (Non Patent Literatures 3 and 4).

As described above, the bacterial lipases of the *Proteus*/*Yersinia* clade have excellent potential as cleansing enzymes in both suitability for cleansing and low-cost manufacturing possibility.

Lipases catalyze various reactions, such as ester synthesis reaction, transesterification reaction, and acidolysis, in addition to ester hydrolysis reaction. Using these reactivities, lipases are used in various applications, such as cleansing, optical resolution, biofuel manufacturing, fat/oil processing, wastewater treatment, pulp manufacturing, and leather manufacturing. Non Patent Literatures 5 and 6 show excellent performance in ester synthesis applications of LipC12 (with the same sequence as Lipr138), and the bacterial lipases of the *Proteus*/*Yersinia* clade are expected to be useful not only for cleaning but also in a variety of industrial applications.

The substrate specificities such as position specificity of an ester bond in acyl glycerol, fatty acid chain length specificity, and enantioselectivity in optical resolution largely vary depending on the type of the lipase. Differences in these substrate specificities are observed even in lipases having closely related sequences, and it is necessary to screen lipases for each reaction system in order to perform an optimum reaction. Accordingly, it is necessary to broaden the sequence diversity of lipases that can be used industrially.

(Patent Literature 1) JP-A-2015-523078
(Patent Literature 2) WO 2020/046613
(Patent Literature 3) JP-A-2015-525248
(Non Patent Literature 1) Hobson, Audrey H., et al., Proceedings of the National Academy of Sciences, 90.12 (1993): 5682-5686
(Non Patent Literature 2) Quyen, ThiDinh, ChiHai Vu, and GiangThi Thu Le, Microbial cell factories, 11.1 (2012): 1-12
(Non Patent Literature 3) Lee, Hong-Weon, et al., Biotechnology letters, 22.19 (2000): 1543-1547
(Non Patent Literature 4) Glogauer, Arnaldo, et al., Microbial cell factories, 10.1 (2011): 1-15
(Non Patent Literature 5) Madalozzo, Aline Dutra, et al., Journal of molecular catalysis b: enzymatic, 116 (2015): 45-51
(Non Patent Literature 6) Madalozzo, Aline Dutra, et al., Biocatalysis and Agricultural Biotechnology, 8 (2016): 294-300

### Summary of the Invention

The present invention relates to the following 1) to 11).
1) A lipase mutant selected from the following (a) to (v):
   (a) a lipase mutant consisting of an amino acid sequence having an identity of at least 82% with the amino acid sequence of SEQ ID NO: 2 and having D at a position corresponding to position 118 of the amino acid sequence of SEQ ID NO: 2;
   (b) a lipase mutant consisting of an amino acid sequence having an identity of at least 80% with the amino acid sequence of SEQ ID NO: 4 and having Y at a position corresponding to position 31 of the amino acid sequence of SEQ ID NO: 2;
   (c) a lipase mutant consisting of an amino acid sequence having an identity of at least 91% with the amino acid sequence of SEQ ID NO: 4 and having G at a position corresponding to position 32 of the amino acid sequence of SEQ ID NO: 2;
   (d) a lipase mutant consisting of an amino acid sequence having an identity of at least 80% with the amino acid sequence of SEQ ID NO: 4 and having C at a position corresponding to position 90 of the amino acid sequence of SEQ ID NO: 2;
   (e) a lipase mutant consisting of an amino acid sequence having an identity of at least 94% with the amino acid sequence of SEQ ID NO: 4 and having L at a position corresponding to position 181 of the amino acid sequence of SEQ ID NO: 2;
   (f) a lipase mutant consisting of an amino acid sequence having an identity of at least 80% with the amino acid sequence of SEQ ID NO: 6 and having A, T, H, or G at a position corresponding to position 222 of the amino acid sequence of SEQ ID NO: 2;
   (g) a lipase mutant consisting of an amino acid sequence having an identity of at least 80% with the amino acid sequence of SEQ ID NO: 8 and having K at a position corresponding to position 39 of the amino acid sequence of SEQ ID NO: 2;
   (h) a lipase mutant consisting of an amino acid sequence having an identity of at least 80% with the amino acid sequence of SEQ ID NO: 8 and having T at a position corresponding to position 125 of the amino acid sequence of SEQ ID NO: 2;
   (i) a lipase mutant consisting of an amino acid sequence having an identity of at least 80% with the amino acid sequence of SEQ ID NO: 8 and having P at a position corresponding to position 126 of the amino acid sequence of SEQ ID NO: 2;
   (j) a lipase mutant consisting of an amino acid sequence having an identity of at least 80% with the amino acid sequence of SEQ ID NO: 8 and having G at a position corresponding to position 293 of the amino acid sequence of SEQ ID NO: 2;
   (k) a lipase mutant consisting of an amino acid sequence having an identity of at least 80% with the amino acid sequence of SEQ ID NO: 10 and having Y at a position corresponding to position 29 of the amino acid sequence of SEQ ID NO: 2;
   (l) a lipase mutant consisting of an amino acid sequence having an identity of at least 80% with the amino acid sequence of SEQ ID NO: 10 and having Y at a position corresponding to position 31 of the amino acid sequence of SEQ ID NO: 2;
   (m) a lipase mutant consisting of an amino acid sequence having an identity of at least 80% with the amino acid sequence of SEQ ID NO: 10 and having G at a position corresponding to position 32 of the amino acid sequence of SEQ ID NO: 2;
   (n) a lipase mutant consisting of an amino acid sequence having an identity of at least 80% with the amino acid sequence of SEQ ID NO: 10 and having G at a position corresponding to position 186 of the amino acid sequence of SEQ ID NO: 2;
   (o) a lipase mutant consisting of an amino acid sequence having an identity of at least 80% with the amino acid sequence of SEQ ID NO: 12 and having Q at a position corresponding to position 206 of the amino acid sequence of SEQ ID NO: 2;
   (p) a lipase mutant consisting of an amino acid sequence having an identity of at least 80% with the19 amino acid sequence of SEQ ID NO: 14 and having Y at a position corresponding to position 31 of the amino acid sequence of SEQ ID NO: 2;
   (q) a lipase mutant consisting of an amino acid sequence having an identity of at least 80% with the amino acid sequence of SEQ ID NO: 14 and having G at a position corresponding to position 32 of the amino acid sequence of SEQ ID NO: 2;
   (r) a lipase mutant consisting of an amino acid sequence having an identity of at least 97% with the amino acid sequence of SEQ ID NO: 14 and having K at a position corresponding to position 77 of the amino acid sequence of SEQ ID NO: 2;
   (s) a lipase mutant consisting of an amino acid sequence having an identity of at least 83% with the amino acid sequence of SEQ ID NO: 14 and having L at a position corresponding to position 181 of the amino acid sequence of SEQ ID NO: 2;
   (t) a lipase mutant consisting of an amino acid sequence having an identity of at least 80% with the amino acid sequence of SEQ ID NO: 16 and having C at a position corresponding to position 90 of the amino acid sequence of SEQ ID NO: 2;
   (u) a lipase mutant consisting of an amino acid sequence having an identity of at least 81% with the amino acid sequence of SEQ ID NO: 16 and having L at a position corresponding to position 181 of the amino acid sequence of SEQ ID NO: 2; and
   (v) a lipase mutant consisting of an amino acid sequence having an identity of at least 80% with the amino acid sequence of SEQ ID NO: 34 and having Y at a position corresponding to position 29 of the amino acid sequence of SEQ ID NO: 2.
2) A lipase mutant selected from the following (aa) to (ae):
   (aa) a lipase mutant consisting of an amino acid sequence having an identity of at least 80% with the amino acid sequence of SEQ ID NO: 4 and having at least two amino acid residues selected from Y at a position corresponding to position 31, G at a position corresponding to position 32, C at a position corresponding to position 90, and L at a position corresponding to position 181 of the amino acid sequence of SEQ ID NO: 2;
   (ab) a lipase mutant consisting of an amino acid sequence having an identity of at least 80% with the amino acid sequence of SEQ ID NO: 8 and having at least two amino acid residues selected from K at a position corresponding to position 39, T at a position corresponding to position 125, P at a position corresponding to position 126, and G at a position corresponding to position 293 of the amino acid sequence of SEQ ID NO: 2;
   (ac) a lipase mutant consisting of an amino acid sequence having an identity of at least 80% with the amino acid sequence of SEQ ID NO: 10 and having at least two amino acid residues selected from Y at a position corresponding to position 29, Y at a position corresponding to position 31, G at a position corresponding to position 32, and G at a position corresponding to position 186 of the amino acid sequence of SEQ ID NO: 2;
   (ad) a lipase mutant consisting of an amino acid sequence having an identity of at least 83% with the amino acid sequence of SEQ ID NO: 14 and having at least two amino acid residues selected from Y at a position corresponding to position 31, G at a position corresponding to position 32, K at a position corresponding to position 77, and L at a position corresponding to position 181 of the amino acid sequence of SEQ ID NO: 2; and
   (ae) a lipase mutant consisting of an amino acid sequence having an identity of at least 80% with the amino acid sequence of SEQ ID NO: 16 and having C at a position corresponding to position 90 and L at a position corresponding to position 181 of the amino acid sequence of SEQ ID NO: 2.
3) An enzyme composition comprising the lipase mutant according to 1) or 2).
4) A polynucleotide encoding the lipase mutant according to 1) or 2).
5) A vector or DNA fragment comprising the polynucleotide according to 4).
6) A transformed cell comprising the vector or DNA fragment according to 5).
7) A method for producing a lipase mutant, comprising a step of culturing the transformed cell according to 6).
8) A method for producing a lipase mutant, comprising a step selected from the following (i) to (xxii):
   (i) a step of substituting an amino acid residue at a position corresponding to position 118 of the amino acid sequence of SEQ ID NO: 2 with D in a polypeptide that consists of an amino acid sequence having an identity of at least 82% with the amino acid sequence of SEQ ID NO: 2 and has lipase activity;
   (ii) a step of substituting an amino acid residue at a position corresponding to position 31 of the amino acid sequence of SEQ ID NO: 2 with Y in a polypeptide that consists of an amino acid sequence having an identity of at least 80% with the amino acid sequence of SEQ ID NO: 4 and has lipase activity;
   (iii) a step of substituting an amino acid residue at a position corresponding to position 32 of the amino acid sequence of SEQ ID NO: 2 with G in a polypeptide that consists of an amino acid sequence having an identity of at least 91% with the amino acid sequence of SEQ ID NO: 4 and has lipase activity;
   (iv) a step of substituting an amino acid residue at a position corresponding to position 90 of the amino acid sequence of SEQ ID NO: 2 with C in a polypeptide that consists of an amino acid sequence having an identity of at least 80% with the amino acid sequence of SEQ ID NO: 4 and has lipase activity;
   (v) a step of substituting an amino acid residue at a position corresponding to position 181 of the amino acid sequence of SEQ ID NO: 2 with L in a polypeptide that consists of an amino acid sequence having an identity of at least 94% with the amino acid sequence of SEQ ID NO: 4 and has lipase activity;
   (vi) a step of substituting an amino acid residue at a position corresponding to position 222 of the amino acid sequence of SEQ ID NO: 2 with A, T, H, or G in a polypeptide that consists of an amino acid sequence having an identity of at least 80% with the amino acid sequence of SEQ ID NO: 6 and has lipase activity;
   (vii) a step of substituting an amino acid residue at a position corresponding to position 39 of the amino acid sequence of SEQ ID NO: 2 with K in a polypeptide that consists of an amino acid sequence having an identity of at least 80% with the amino acid sequence of SEQ ID NO: 8 and has lipase activity;
   (viii) a step of substituting an amino acid residue at a position corresponding to position 125 of the amino acid sequence of SEQ ID NO: 2 with T in a polypeptide that consists of an amino acid sequence having an identity of at least 80% with the amino acid sequence of SEQ ID NO: 8 and has lipase activity;
   (ix) a step of substituting an amino acid residue at a position corresponding to position 126 of the amino acid sequence of SEQ ID NO: 2 with P in a polypeptide that consists of an amino acid sequence having an identity of at least 80% with the amino acid sequence of SEQ ID NO: 8 and has lipase activity;
   (x) a step of substituting an amino acid residue at a position corresponding to position 293 of the amino acid sequence of SEQ ID NO: 2 with G in a polypeptide that consists of an amino acid sequence having an identity of at least 80% with the amino acid sequence of SEQ ID NO: 8 and has lipase activity;
   (xi) a step of substituting an amino acid residue at a position corresponding to position 29 of the amino acid sequence of SEQ ID NO: 2 with Y in a polypeptide that consists of an amino acid sequence having an identity of at least 80% with the amino acid sequence of SEQ ID NO: 10 and has lipase activity;
   (xii) a step of substituting an amino acid residue at a position corresponding to position 31 of the amino acid sequence of SEQ ID NO: 2 with Y in a polypeptide that consists of an amino acid sequence having an identity of at least 80% with the amino acid sequence of SEQ ID NO: 10 and has lipase activity;
   (xiii) a step of substituting an amino acid residue at a position corresponding to position 32 of the amino acid sequence of SEQ ID NO: 2 with C in a polypeptide that consists of an amino acid sequence having an identity of at least 80% with the amino acid sequence of SEQ ID NO: 10 and has lipase activity;
   (xiv) a step of substituting an amino acid residue at a position corresponding to position 186 of the amino acid sequence of SEQ ID NO: 2 with G in a polypeptide that consists of an amino acid sequence having an identity of at least 80% with the amino acid sequence of SEQ ID NO: 10 and has lipase activity;
   (xv) a step of substituting an amino acid residue at a position corresponding to position 206 of the amino acid sequence of SEQ ID NO: 2 with Q in a polypeptide that consists of an amino acid sequence having an identity of at least 80% with the amino acid sequence of SEQ ID NO: 12 and has lipase activity;
   (xvi) a step of substituting an amino acid residue at a position corresponding to position 31 of the amino acid sequence of SEQ ID NO: 2 with Y in a polypeptide that consists of an amino acid sequence having an identity of at least 80% with the amino acid sequence of SEQ ID NO: 14 and has lipase activity;
   (xvii) a step of substituting an amino acid residue at a position corresponding to position 32 of the amino acid sequence of SEQ ID NO: 2 with G in a polypeptide that consists of an amino acid sequence having an identity of at least 80% with the amino acid sequence of SEQ ID NO: 14 and has lipase activity;
   (xviii) a step of substituting an amino acid residue at a position corresponding to position 77 of the amino acid sequence of SEQ ID NO: 2 with K in a polypeptide that consists of an amino acid sequence having an identity of at least 97% with the amino acid sequence of SEQ ID NO: 14 and has lipase activity;
   (xix) a step of substituting an amino acid residue at a position corresponding to position 181 of the amino acid sequence of SEQ ID NO: 2 with L in a polypeptide that consists of an amino acid sequence having an identity of at least 83% with the amino acid sequence of SEQ ID NO: 14 and has lipase activity;
   (xx) a step of substituting an amino acid residue at a position corresponding to position 90 of the amino acid sequence of SEQ ID NO: 2 with C in a polypeptide that consists of an amino acid sequence having an identity of at least 80% with the amino acid sequence of SEQ ID NO: 16 and has lipase activity;
   (xxi) a step of substituting an amino acid residue at a position corresponding to position 181 of the amino acid sequence of SEQ ID NO: 2 with L in a polypeptide that consists of an amino acid sequence having an identity of at least 81% with the amino acid sequence of SEQ ID NO: 16 and has lipase activity; and
   (xxii) a step of substituting an amino acid residue at a position corresponding to position 29 of the amino acid sequence of SEQ ID NO: 2 with Y in a polypeptide that consists of an amino acid sequence having an identity of at least 80% with the amino acid sequence of SEQ ID NO: 34 and has lipase activity.
9) A method for preparing a lipase mutant, comprising a step selected from the following (xxiii) to (xxvii):
   (xxiii) a step of substituting at least two amino acid residues at positions selected from positions corresponding to positions 31, 32, 90, and 181 of the amino acid sequence of SEQ ID NO: 2 with Y at a position corresponding to position 31, G at a position corresponding to position 32, C at a position corresponding to position 90, and L at a position corresponding to position 181 in a polypeptide that consists of an amino acid sequence having an identity of at least 80% with the amino acid sequence of SEQ ID NO: 4 and has lipase activity;
   (xxiv) a step of substituting at least two amino acid residues at positions selected from positions corresponding to positions 39, 125, 126, and 293 of the amino acid sequence of SEQ ID NO: 2 with K at a position corresponding to position 39, T at a position corresponding to position 125, P at a position corresponding to position 126, and G at a position corresponding to position 293 in a polypeptide that consists of an amino acid sequence having an identity of at least 80% with the amino acid sequence of SEQ ID NO: 8 and has lipase activity;
   (xxv) a step of substituting at least two amino acid residues at positions selected from positions corresponding to positions 29, 31, 32, and 186 of the amino acid sequence of SEQ ID NO: 2 with Y at a position corresponding to position 29, Y at a position corresponding to position 31, G at a position corresponding to position 32, and G at a position corresponding to position 186 in a polypeptide that consists of an amino acid sequence having an identity of at least 80% with the amino acid sequence of SEQ ID NO: 10 and has lipase activity;
   (xxvi) a step of substituting at least two amino acid residues at positions selected from positions corresponding to positions 31, 32, 77, and 181 of the amino acid sequence of SEQ ID NO: 2 with Y at a position corresponding to position 31, G at a position corresponding to position 32, K at a position corresponding to position 77, and L at a position corresponding to position 181 in a polypeptide that consists of an amino acid sequence having an identity of at least 83% with the amino acid sequence of SEQ ID NO: 14 and has lipase activity; and
   (xxvii) a step of substituting an amino acid residue at a position corresponding to position 90 of the amino acid sequence of SEQ ID NO: 2 with C and an amino acid residue at a position corresponding to position 181 with L in a polypeptide that consists of an amino acid sequence having an identity of at least 80% with the amino acid sequence of SEQ ID NO: 16 and has lipase activity.
10) A method for improving heterologous expression of a lipase, comprising a step selected from the above (i) to (xxii).
11) A method for improving heterologous expression of a lipase, comprising a step selected from the above (xxiii) to (xxvii).

### Brief Description of Drawing

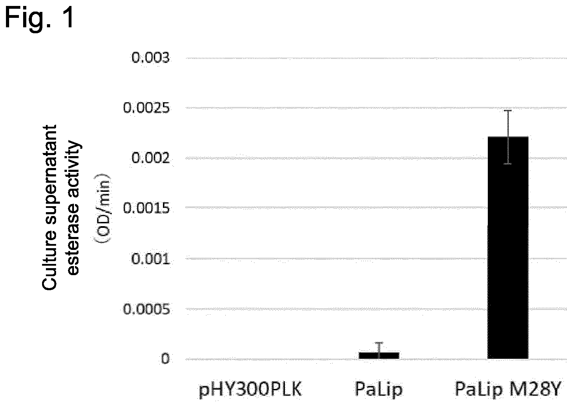

Fig. 1 shows the esterase activity of each lipase. pHY300PLK shows the results of an empty vector that does not encode a lipase.

### Detailed Description of the Invention

All Patent Literatures, Non Patent Literatures, and other publications cited herein are incorporated by reference in their entireties.

As used herein, the term "lipase" refers to triacylglycerol lipase (EC3.1.1.3) and means an enzyme group that has an activity of hydrolyzing an ester bond in a lipid to generate a fatty acid. The lipase activity is typically an esterase activity and can be determined by measuring the rate of increase in absorbance associated with the release of 4-nitrophenol by hydrolysis of 4-nitrophenyl octanoate. A specific procedure of measuring lipase activity will be described in detail in Examples described later.

As used herein, the term *"Proteus*/*Yersinia* clade" refers to a clade including lipases derived from *Proteus* bacteria and *Yersinia* bacteria on a rooted phylogenetic tree showing protein evolution. For example, the amino acid sequences of SEQ ID NOs: 2, 4, 6, 8, 10, 12, 14, 16, 18, 20, 22, 24, 26, 28, 30, and 32 are all amino acid sequences of lipases belonging to the *Proteus*/*Yersinia* clade.

As used herein, the identity between amino acid sequences or nucleotide sequences is calculated by the Lipman-Pearson method (Science, 1985, 227: 1435-1441). Specifically, the identity is calculated by analysis using the homology analysis (Search homology) program of genetic information processing software GENETYX Ver. 12 and setting the Unit size to compare (ktup) to 2.

As used herein, the term "identity of at least 80%" with respect to an amino acid sequence and nucleotide sequence refers to an identity of 80% or more, preferably 85% or more, more preferably 90% or more, further preferably 92% or more, further preferably 94% or more, further preferably 95% or more, further preferably 96% or more, further preferably 98% or more, further preferably 99% or more, and further preferably 99.5% or more. The term "identity of at least 81%" refers to an identity of 81% or more, preferably 85% or more, more preferably 90% or more, further preferably 92% or more, further preferably 94% or more, further preferably 95% or more, further preferably 96% or more, further preferably 98% or more, further preferably 99% or more, and further preferably 99.5% or more. The term "identity of at least 83%" refers to an identity of 83% or more, preferably 85% or more, more preferably 90% or more, further preferably 92% or more, further preferably 94% or more, further preferably 95% or more, further preferably 96% or more, further preferably 98% or more, further preferably 99% or more, and further preferably 99.5% or more. The term "identity of at least 91%" refers to an identity of 91% or more, preferably 92% or more, more preferably 94% or more, further preferably 95% or more, further preferably 96% or more, further preferably 98% or more, further preferably 99% or more, and further preferably 99.5% or more. The term "identity of at least 94%" refers to an identity of 94% or more, preferably 95% or more, more preferably 96% or more, further preferably 98% or more, further preferably 99% or more, and further preferably 99.5% or more. The term "identity of at least 97%" refers to an identity of 97% or more, more preferably 98% or more, further preferably 99% or more, and further preferably 99.5% or more.

As used herein, a "corresponding position" on an amino acid sequence or nucleotide sequence can be determined by aligning a target sequence and a reference sequence (for example, the amino acid sequence of SEQ ID NO: 2) so as to give the maximum homology. The alignment of amino acid sequences or nucleotide sequences can be carried out using a known algorithm, and the procedure thereof is known to those skilled in the art. For example, alignment can be performed by using Clustal W multiple alignment program (Thompson, J.D., et al., 1994, Nucleic Acids Res., 22: 4673-4680) with the default set. Alternatively, Clustal W2 or Clustal omega, which are revised editions of Clustal W, can also be used. Clustal W, Clustal W2, and Clustal omega can be used on the website of, for example, the Clustal [www.clustal.org] operated by University College Dublin, the European Bioinformatics Institute: EBI [www.ebi.ac.uk/index.html], or the DNA data bank of Japan (DDBJ [www.ddbj.nig.ac.jp/searches-j.html]) operated by the National Institute of Genetics. A position in a target sequence aligned at any position in a reference sequence by the above-described alignment is considered to be a "position corresponding" to the position.

Those skilled in the art can further finely adjust and optimize the alignment of the amino acid sequence obtained above. The optimum alignment is preferably determined by considering the similarity between amino acid sequences, the frequency of gaps to be inserted, and so on. Here, the similarity between amino acid sequences refers to, when two amino acid sequences are aligned, the ratio (%) of the number of positions where the same or similar amino acid residues are present in both the sequences to the number of the full-length amino acid residues. Similar amino acid residues mean that the amino acid residues have similar properties in the polarity or electric charge to each other and cause so-called conservative substitution among 20 amino acids constituting a protein. Groups consisting of such similar amino acid residues are well known to those skilled in the art, and examples thereof include, but not limited to, arginine and lysine or glutamine; glutamic acid and aspartic acid or glutamine; serine and threonine or alanine; glutamine and asparagine or arginine; and leucine and isoleucine.

As used herein, the term "amino acid residue" means 20 amino acid residues constituting a protein: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

As used herein, an amino acid position and mutant are designated using the officially recognized IUPAC one-letter amino acid abbreviation as follows.

An amino acid at a predetermined position is designated by [amino acid, position]. For example, phenylalanine at position 31 is shown as "F31".

"Substitution" of an amino acid is designated by [original amino acid, position, substituted amino acid]. For example, substitution of phenylalanine at position 31 with tyrosine is designated as "F31Y".

When different modifications can be introduced at one position, the different modifications are separated by a slash ("/"). For example, "I222A/T/H/G" represents substitution of isoleucine at position 222 with alanine, threonine, histidine, or glycine.

As used herein, the term "operable linkage" between a regulatory region, such as a promoter, and a gene indicates that a gene and a regulatory region are linked such that the gene can be expressed under the control of the regulatory region. The procedure of "operable linkage" between a gene and a regulatory region is known to those skilled in the art.

As used herein, the term "upstream" and "downstream" with respect to a gene refer to the upstream and downstream of the gene in the transcription direction. For example, "a gene located downstream of a promoter" means that the gene is present on the 3' side of the promoter in a DNA sense strand, and upstream of a gene means a region on the 5' side of the gene in a DNA sense strand.

As used herein, the "parent" polypeptide of a given mutant polypeptide refers to a polypeptide that brings about the mutant polypeptide by a predetermined mutation of an amino acid residue. In other words, the "parent" polypeptide is a polypeptide before the mutation is introduced into the mutant polypeptide. Such a parent polypeptide can be a natural (wild-type) polypeptide or its mutant.

The present inventers evaluated heterologous expression in *Bacillus subtilis* regarding 16 various lipases belonging to the *Proteus*/*Yersinia* clade that includes lipases derived from *Proteus* bacteria and *Yersinia* bacteria on a rooted phylogenetic tree showing protein evolution, and found for the first time a problem: although lipases of the *Proteus*/*Yersinia* clade have been reported to have advantages in heterologous expression, clear expression was observed in three lipases only, and the diversity of sequences that can be heterologously expressed is actually small in lipases of the *Proteus*/*Yersinia* clade. Accordingly, the present invention relates to a provision of a lipase mutant having improved heterologous expression.

The present inventors intensively studied in view of the above-mentioned problem and as a result, found mutations that improve the heterologous expression of lipases by using 8 lipases among the above-mentioned 16 lipases and one lipase closely related to them.

The lipase mutant of the present invention has improved heterologous expression compared to the parent lipase. Such a lipase mutant can be produced inexpensively by heterologous expression, and can be used not only in cleansing applications but also in general industrial applications such as optical resolution, biofuel manufacturing, fat/oil processing, wastewater treatment, pulp manufacturing, and leather manufacturing.

### <1. Lipase mutant and method for produced it>

The present invention provides a lipase mutant with improved heterologous expression and a method for preparing it.

In one aspect, the present invention provides a method for produced a lipase mutant. The method of the present invention includes substitution of an amino acid residue at a predetermined position numbered according to SEQ ID NO: 2 with a predetermined amino acid residue in a parent lipase.

An example of the parent lipase of the lipase mutant of the present invention is a polypeptide that consists of an amino acid sequence having an identity of at least 82% with the amino acid sequence of SEQ ID NO: 2 and has lipase activity. Here, the polypeptide consisting of the amino acid sequence of SEQ ID NO: 2 and having lipase activity is lipase SspLip derived from *Serratia* sp. (NCBI Accession No. WP_025122441.1). The parent lipase that is a polypeptide consisting of an amino acid sequence having an identity of at least 82% with the amino acid sequence of SEQ ID NO: 2 and having lipase activity preferably has E at a position corresponding to position 118 of the amino acid sequence of SEQ ID NO: 2.

When the lipase mutant of the present invention is produced from the parent lipase, in the parent lipase, the amino acid residue at a position corresponding to position 118 of the amino acid sequence of SEQ ID NO: 2 is substituted with D.

When the lipase mutant of the present invention is produced from the parent lipase, in the parent lipase, more specifically, E at a position corresponding to position 118 of the amino acid sequence of SEQ ID NO: 2 is substituted with D.

Another example of the parent lipase is a polypeptide that consists of an amino acid sequence having an identity of at least 80%, 91%, or 94% with the amino acid sequence of SEQ ID NO: 4 and has lipase activity. Here, the polypeptide consisting of the amino acid sequence of SEQ ID NO: 4 and having lipase activity is lipase PfLip (NCBI Accession No. WP_123598507.1) derived from *Pseudomonas frederiksbergensis.* The parent lipase that is a polypeptide consisting of an amino acid sequence having at least the above predetermined identity with the amino acid sequence of SEQ ID NO: 4 and having lipase activity preferably has F at a position corresponding to position 31 of the amino acid sequence of SEQ ID NO: 2, E at a position corresponding to position 32 of the amino acid sequence of SEQ ID NO: 2, A at a position corresponding to position 90 of the amino acid sequence of SEQ ID NO: 2, or V at a position corresponding to position 181 of the amino acid sequence of SEQ ID NO: 2, more preferably has F and E at positions corresponding to positions 31 and 32, respectively, of the amino acid sequence of SEQ ID NO: 2, A and V at positions corresponding to positions 90 and 181, respectively, of the amino acid sequence of SEQ ID NO: 2, or E and A at positions corresponding to positions 32 and 90, respectively, of the amino acid sequence of SEQ ID NO: 2, and further preferably has F, E, A, and V at positions corresponding to positions 31, 32, 90, and 181, respectively, of the amino acid sequence of SEQ ID NO: 2.

When the lipase mutant of the present invention is produced from the parent lipase, in the parent lipase, preferably, the amino acid residue at the opposition corresponding to position 31 of the amino acid sequence of SEQ ID NO: 2 is substituted with Y, the amino acid residue at a position corresponding to position 32 of the amino acid sequence of SEQ ID NO: 2 is substituted with G, the amino acid residue at a position corresponding to position 90 of the amino acid sequence of SEQ ID NO: 2 is substituted with C, the amino acid residue at a position corresponding to position 181 of the amino acid sequence of SEQ ID NO: 2 is substituted with L, or at least two (preferably three, more preferably four) amino acid residues at positions selected from positions corresponding to positions 31, 32, 90, and 181 of the amino acid sequence of SEQ ID NO: 2 are substituted with Y at a position corresponding to position 31, G at a position corresponding to position 32, C at a position corresponding to position 90, and L at a position corresponding to position 181. In the parent lipase, more preferably, the amino acid residue at a position corresponding to position 32 of the amino acid sequence of SEQ ID NO: 2 is substituted with G, the amino acid residue at a position corresponding to position 90 of the amino acid sequence of SEQ ID NO: 2 is substituted with C, the amino acid residue at a position corresponding to position 181 of the amino acid sequence of SEQ ID NO: 2 is substituted with L, the amino acid residues at positions corresponding to positions 31 and 32 of the amino acid sequence of SEQ ID NO: 2 are respectively substituted with Y and G, the amino acid residues at positions corresponding to positions 90 and 181 of the amino acid sequence of SEQ ID NO: 2 are respectively substituted with C and L, the amino acid residues at positions corresponding to positions 32 and 90 of the amino acid sequence of SEQ ID NO: 2 are respectively substituted with G and C, or the amino acid residues at positions corresponding to positions 31, 32, 90, and 181 of the amino acid sequence of SEQ ID NO: 2 are respectively substituted with Y, G, C, and L.

When the lipase mutant of the present invention is produced from the parent lipase, more specifically, in the parent lipase, preferably, F at a position corresponding to position 31 of the amino acid sequence of SEQ ID NO: 2 is substituted with Y; E at a position corresponding to position 32 of the amino acid sequence of SEQ ID NO: 2 is substituted with G; A at a position corresponding to position 90 of the amino acid sequence of SEQ ID NO: 2 is substituted with C; V at a position corresponding to position 181 of the amino acid sequence of SEQ ID NO: 2 is substituted with L; or at least two (preferably three, more preferably four) amino acid residues selected from F at a position corresponding to position 31, E at a position corresponding to position 32, A at a position corresponding to position 90, and V at a position corresponding to position 181 of the amino acid sequence of SEQ ID NO: 2 are substituted with Y at a position corresponding to position 31, G at a position corresponding to position 32, C at a position corresponding to position 90, and L at a position corresponding to position 181. In the parent lipase, more preferably, E at a position corresponding to position 32 of the amino acid sequence of SEQ ID NO: 2 is substituted with G; A at a position corresponding to position 90 of the amino acid sequence of SEQ ID NO: 2 is substituted with C; V at a position corresponding to position 181 of the amino acid sequence of SEQ ID NO: 2 is substituted with L; F and E at positions corresponding to positions 31 and 32 of the amino acid sequence of SEQ ID NO: 2 are respectively substituted with Y and **G;** A and V at positions corresponding to positions 90 and 181 of the amino acid sequence of SEQ ID NO: 2 are respectively substituted with C and L; E and A at positions corresponding to positions 32 and 90 of the amino acid sequence of SEQ ID NO: 2 are respectively substituted with G and C; or F, E, A, and V at positions corresponding to positions 31, 32, 90, and 181 of the amino acid sequence of SEQ ID NO: 2 are respectively substituted with Y, G, C, and L.

Another example of the parent lipase is a polypeptide that consists of an amino acid having an identity of at least 80% with the amino acid sequence of SEQ ID NO: 6 and has lipase activity. Here, the polypeptide consisting of the amino acid sequence of SEQ ID NO: 6 and having lipase activity is lipase EtLip (NCBI Accession No. WP_115457195.1) derived from *Enterobacillus tribolii.* The parent lipase that is a polypeptide consisting of an amino acid sequence having an identity of at least 80% with the amino acid sequence of SEQ ID NO: 6 and having lipase activity preferably has I at a position corresponding to position 222 of the amino acid sequence of SEQ ID NO: 2.

When the lipase mutant of the present invention is produced from the parent lipase, in the parent lipase, the amino acid residue at a position corresponding to position 222 of the amino acid sequence of SEQ ID NO: 2 is substituted with A, T, H, or G.

When the lipase mutant of the present invention is produced from the parent lipase, in the parent lipase, more specifically, I at a position corresponding to position 222 of the amino acid sequence of SEQ ID NO: 2 is substituted with A, T, H, or G.

Another example of the parent lipase is a polypeptide that consists of an amino acid sequence having an identity of at least 80% with the amino acid sequence of SEQ ID NO: 8 and has lipase activity. Here, the polypeptide consisting of the amino acid sequence of SEQ ID NO: 8 and having lipase activity is lipase YeLip (NCBI Accession No. WP_064517898.1) derived from *Yersinia entomophaga.* The parent lipase that is a polypeptide consisting of an amino acid sequence having an identity of at least 80% with the amino acid sequence of SEQ ID NO: 8 and having lipase activity preferably has V at a position corresponding to position 39 of the amino acid sequence of SEQ ID NO: 2, V at a position corresponding to position 125 of the amino acid sequence of SEQ ID NO: 2, E at a position corresponding to position 126 of the amino acid sequence of SEQ ID NO: 2, or S at a position corresponding to position 293 of the amino acid sequence of SEQ ID NO: 2, more preferably has V and E at positions corresponding to positions 125 and 126, respectively, of the amino acid sequence of SEQ ID NO: 2 or V and S at positions corresponding to positions 39 and 293, respectively, of the amino acid sequence of SEQ ID NO: 2, further preferably has V, E, and S at positions corresponding to positions 125, 126, and 293, respectively, of the amino acid sequence of SEQ ID NO: 2, V, V, and E at positions corresponding to positions 39, 125, and 126 of the amino acid sequence of SEQ ID NO: 2, or V, E, and S at positions corresponding to positions 39, 126, and 293, respectively, of the amino acid sequence of SEQ ID NO: 2, and further preferably has V, V, E, and S at positions corresponding to positions 39, 125, 126, and 293, respectively, of the amino acid sequence of SEQ ID NO: 2.

When the lipase mutant of the present invention is produced from the parent lipase, in the parent lipase, preferably, the amino acid residue at a position corresponding to position 39 of the amino acid sequence of SEQ ID NO: 2 is substituted with K, the amino acid residue at a position corresponding to position 125 of the amino acid sequence of SEQ ID NO: 2 is substituted with T, the amino acid residue at a position corresponding to position 126 of the amino acid sequence of SEQ ID NO: 2 is substituted with P, the amino acid residue at a position corresponding to position 293 of the amino acid sequence of SEQ ID NO: 2 is substituted with G, or at least two (preferably three, more preferably four) amino acid residues at positions selected from positions corresponding to positions 39, 125, 126, and 293 of the amino acid sequence of SEQ ID NO: 2 are substituted with K at a position corresponding to position 39, T at a position corresponding to position 125, P at a position corresponding to position 126, and G at a position corresponding to position 293. In the parent lipase, more preferably, the amino acid residue at a position corresponding to position 39 of the amino acid sequence of SEQ ID NO: 2 is substituted with K, the amino acid residue at a position corresponding to position 126 of the amino acid sequence of SEQ ID NO: 2 is substituted with P, the amino acid residue at a position corresponding to position 293 of the amino acid sequence of SEQ ID NO: 2 is substituted with G, the amino acid residues at positions corresponding to positions 125 and 126 of the amino acid sequence of SEQ ID NO: 2 are respectively substituted with T and P, the amino acid residues at positions corresponding to positions 39 and 293 of the amino acid sequence of SEQ ID NO: 2 are respectively substituted with K and G, the amino acid residues at positions corresponding to positions 125, 126, and 293 of the amino acid sequence of SEQ ID NO: 2 are respectively substituted with T, P, and G, the amino acid residues at positions corresponding to positions 39, 125, and 126 of the amino acid sequences of SEQ ID NO: 2 are respectively substituted with K, T, and P, the amino acid residues at positions corresponding to positions 39, 126, and 293 of the amino acid sequence of SEQ ID NO: 2 are respectively substituted with K, P, and G, or the amino acid residues at positions corresponding to positions 39, 125, 126, and 293 of the amino acid sequence of SEQ ID NO: 2 are respectively substituted with K, T, P, and G.

When the lipase mutant of the present invention is produced from the parent lipase, more specifically, in the parent lipase, preferably, V at a position corresponding to position 39 of the amino acid sequence of SEQ ID NO: 2 is substituted with K; V at a position corresponding to position 125 of the amino acid sequence of SEQ ID NO: 2 is substituted with T; E at a position corresponding to position 126 of the amino acid sequence of SEQ ID NO: 2 is substituted with P; S at a position corresponding to position 293 of the amino acid sequence of SEQ ID NO: 2 is substituted with G; or at least two (preferably three, more preferably four) amino acid residues selected from V at a position corresponding to position 39, V at a position corresponding to position 125, E at a position corresponding to position 126, and S at a position corresponding to position 293 of the amino acid sequence of SEQ ID NO: 2 are substituted with K at a position corresponding to position 39, T at a position corresponding to position 125, P at a position corresponding to position 126, and G at a position corresponding to position 293. In the parent lipase, more preferably, V at a position corresponding to position 39 of the amino acid sequence of SEQ ID NO: 2 is substituted with K; E at a position corresponding to position 126 of the amino acid sequence of SEQ ID NO: 2 is substituted with P; S at a position corresponding to position 293 of the amino acid sequence of SEQ ID NO: 2 is substituted with G; V and E at positions corresponding to positions 125 and 126 of the amino acid sequence of SEQ ID NO: 2 are respectively substituted with T and P; V and S at positions corresponding to positions 39 and 293 of the amino acid sequence of SEQ ID NO: 2 are respectively substituted with K and G; V, E, and S at positions corresponding to positions 125, 126, and 293 of the amino acid sequence of SEQ ID NO: 2 are respectively substituted with T, P, and G; V, V, and E at positions corresponding to positions 39, 125, and 126 of the amino acid sequence of SEQ ID NO: 2 are respectively substituted with K, T, and P; V, E, and S at positions corresponding to positions 39, 126, and 293 of the amino acid sequence of SEQ ID NO: 2 are respectively substituted with K, P, and G; or V, V, E, and S at positions corresponding to positions 39, 125, 126, and 293 of the amino acid sequence of SEQ ID NO: 2 are respectively substituted with K, T, P, and G.

Another example of the parent lipase is a polypeptide that consists of an amino acid sequence having an identity of at least 80% with the amino acid sequence of SEQ ID NO: 10 and has lipase activity. Here, the polypeptide consisting of the amino acid sequence of SEQ ID NO: 10 and having lipase activity is lipase AnLip (NCBI Accession No. WP_026822497.1) derived from *Arsenophonus nasoniae.* The parent lipase that is a polypeptide consisting of an amino acid sequence having an identity of at least 80% with the amino acid sequence of SEQ ID NO: 10 and having lipase activity preferably has C at a position corresponding to position 29 of the amino acid sequence of SEQ ID NO: 2, F at a position corresponding to position 31 of the amino acid sequence of SEQ ID NO: 2, A at a position corresponding to position 32 of the amino acid sequence of SEQ ID NO: 2, or R at a position corresponding to position 186 of the amino acid sequence of SEQ ID NO: 2, more preferably has C and R at positions corresponding to positions 29 and 186, respectively, of the amino acid sequence of SEQ ID NO: 2, and further preferably has C, F, A, and R at positions corresponding to positions 29, 31, 32, and 186 of the amino acid sequence of SEQ ID NO: 2.

When the lipase mutant of the present invention is produced from the parent lipase, in the parent lipase, preferably, the amino acid residue at a position corresponding to position 29 of the amino acid sequence of SEQ ID NO: 2 is substituted with Y, the amino acid residue at a position corresponding to position 31 of the amino acid sequence of SEQ ID NO: 2 is substituted with Y, the amino acid residue at a position corresponding to position 32 of the amino acid sequence of SEQ ID NO: 2 is substituted with G, at least two (preferably three, more preferably four) amino acid residue at a position corresponding to position 186 of the amino acid sequence of SEQ ID NO: 2 is substituted with G, or the amino acid residues at positions selected from positions corresponding to positions 29, 31, 32, and 186 of the amino acid sequence of SEQ ID NO: 2 are substituted with Y at a position corresponding to position 29, Y at a position corresponding to position 31, G at a position corresponding to position 32, and G at a position corresponding to position 186. More preferably, in the parent lipase, the amino acid residue at a position corresponding to position 29 of the amino acid sequence of SEQ ID NO: 2 is substituted with Y, the amino acid residue at a position corresponding to position 186 of the amino acid sequence of SEQ ID NO: 2 is substituted with G, the amino acid residues at positions corresponding to positions 29 and 186 of the amino acid sequence of SEQ ID NO: 2 are respectively substituted with Y and G, or the amino acid residues at positions corresponding to positions 29, 31, 32, and 186 of the amino acid sequence of SEQ ID NO: 2 are respectively substituted with Y, Y, G, and G.

When the lipase mutant of the present invention is produced from the parent lipase, more specifically, in the parent lipase, preferably, C at a position corresponding to position 29 of the amino acid sequence of SEQ ID NO: 2 is substituted with Y; F at a position corresponding to position 31 of the amino acid sequence of SEQ ID NO: 2 is substituted with Y; A at a position corresponding to position 32 of the amino acid sequence of SEQ ID NO: 2 is substituted with G; R at a position corresponding to position 186 of the amino acid sequence of SEQ ID NO: 2 is substituted with G; or at least two (preferably three, more preferably four) amino acid residues selected from C at a position corresponding to position 29, F at a position corresponding to position 31, A at a position corresponding to position 32, and R at a position corresponding to position 186 of the amino acid sequence of SEQ ID NO: 2 are substituted with Y at a position corresponding to position 29, Y at a position corresponding to position 31, G at a position corresponding to position 32, and G at a position corresponding to position 186. More preferably, in the parent lipase, C at a position corresponding to position 29 of the amino acid sequence of SEQ ID NO: 2 is substituted with Y; R at a position corresponding to position 186 of the amino acid sequence of SEQ ID NO: 2 is substituted with G; C and R at positions corresponding to positions 29 and 186 of the amino acid sequence of SEQ ID NO: 2 are respectively substituted with Y and G, or C, F, A, and R at positions corresponding to positions 29, 31, 32, and 186 of the amino acid sequence of SEQ ID NO: 2 are respectively substituted with Y, Y, G, and G.

An example of the parent lipase of the lipase mutant of the present invention is a polypeptide that consists of an amino acid sequence having an identity of at least 80% with the amino acid sequence of SEQ ID NO: 12 and has lipase activity. Here, the polypeptide consisting of the amino acid sequence of SEQ ID NO: 12 and having lipase activity is lipase YfLip (NCBI Accession No. WP_145530745.1) derived from *Yersinia frederiksenii.* The parent lipase that is a polypeptide consisting of an amino acid sequence having an identity of at least 80% with the amino acid sequence of SEQ ID NO: 12 and having lipase activity preferably has G at a position corresponding to position 206 of the amino acid sequence of SEQ ID NO: 2.

When the lipase mutant of the present invention is produced from the parent lipase, in the parent lipase, the amino acid residue at a position corresponding to position 206 of the amino acid sequence of SEQ ID NO: 2 is substituted with Q.

When the lipase mutant of the present invention is produced from the parent lipase, in the parent lipase, more specifically, G at a position corresponding to position 206 of the amino acid sequence of SEQ ID NO: 2 is substituted with Q.

Another example of the parent lipase is a polypeptide that consists of an amino acid sequence having an identity of at least 80%, 83%, or 97% with the amino acid sequence of SEQ ID NO: 14 and has lipase activity. Here, the polypeptide consisting of the amino acid sequence of SEQ ID NO: 14 and having lipase activity is lipase PspLip (NCBI Accession No. WP_056840927.1) derived from *Pseudomonas* sp. The parent lipase that is a polypeptide consisting of an amino acid sequence having at least the above predetermined identity with the amino acid sequence of SEQ ID NO: 14 and having lipase activity preferably has F at a position corresponding to position 31 of the amino acid sequence of SEQ ID NO: 2, D at a position corresponding to position 32 of the amino acid sequence of SEQ ID NO: 2, R at a position corresponding to position 77 of the amino acid sequence of SEQ ID NO: 2, or M at a position corresponding to position 181 of the amino acid sequence of SEQ ID NO: 2, more preferably has F and D at positions corresponding to positions 31 and 32, respectively, of the amino acid sequence of SEQ ID NO: 2 or R and M at positions corresponding to positions 77 and 181, respectively, of the amino acid sequence of SEQ ID NO: 2, further preferably has F, D, and M at positions corresponding to positions 31, 32, and 181, respectively, of the amino acid sequence of SEQ ID NO: 2, and further preferably has F, D, R, and M at positions corresponding to positions 31, 32, 77, and 181, respectively, of the amino acid sequence of SEQ ID NO: 2.

When the lipase mutant of the present invention is produced from the parent lipase, in the parent lipase, preferably, the amino acid residue at a position corresponding to position 31 of the amino acid sequence of SEQ ID NO: 2 is substituted with Y, the amino acid residue at a position corresponding to position 32 of the amino acid sequence of SEQ ID NO: 2 is substituted with G, the amino acid residue at a position corresponding to position 77 of the amino acid sequence of SEQ ID NO: 2 is substituted with K, the amino acid residue at a position corresponding to position 181 of the amino acid sequence of SEQ ID NO: 2 is substituted with L, or at least two (preferably three, more preferably four) amino acid residues at positions selected from positions corresponding to positions 31, 32, 77, and 181 of the amino acid sequence of SEQ ID NO: 2 are substituted with Y at a position corresponding to position 31, G at a position corresponding to position 32, K at a position corresponding to position 77, and L at a position corresponding to position 181. In the parent lipase, more preferably, the amino acid residue at a position corresponding to position 77 of the amino acid sequence of SEQ ID NO: 2 is substituted with K, the amino acid residues at positions corresponding to positions 31 and 32 of the amino acid sequence of SEQ ID NO: 2 are respectively substituted with Y and G, the amino acid residues at positions corresponding to positions 77 and 181 of the amino acid sequence of SEQ ID NO: 2 are respectively substituted with K and L, the amino acid residues at positions corresponding to positions 31, 32, and 181 of the amino acid sequence of SEQ ID NO: 2 are respectively substituted with Y, G, and L, or the amino acid residues at positions corresponding to positions 31, 32, 77, and 181 of the amino acid sequence of SEQ ID NO: 2 are respectively substituted with Y, G, K, and L.

When the lipase mutant of the present invention is produced from the parent lipase, more specifically, in the parent lipase, preferably, F at a position corresponding to position 31 of the amino acid sequence of SEQ ID NO: 2 is substituted with Y; D at a position corresponding to position 32 of the amino acid sequence of SEQ ID NO: 2 is substituted with G; R at a position corresponding to position 77 of the amino acid sequence of SEQ ID NO: 2 is substituted with K; M at a position corresponding to position 181 of the amino acid sequence of SEQ ID NO: 2 is substituted with L; or at least two (preferably three, more preferably four) amino acid residues selected from F at a position corresponding to position 31, D at a position corresponding to position 32, R at a position corresponding to position 77, and M at a position corresponding to position 181 of the amino acid sequence of SEQ ID NO: 2 are substituted with Y at a position corresponding to position 31, G at a position corresponding to position 32, K at a position corresponding to position 77, and L at a position corresponding to position 181. In the parent lipase, more preferably, R at a position corresponding to position 77 of the amino acid sequence of SEQ ID NO: 2 is substituted with K; F and D at positions corresponding to positions 31 and 32 of the amino acid sequence of SEQ ID NO: 2 are respectively substituted with Y and G; R and M at positions corresponding to positions 77 and 181 of the amino acid sequence of SEQ ID NO: 2 are respectively substituted with K and L; F, D, and M at positions corresponding to positions 31, 32, and 181 of the amino acid sequence of SEQ ID NO: 2 are respectively substituted with Y, G, and L; or F, D, R, and M at positions corresponding to positions 31, 32, 77, and 181 of the amino acid sequence of SEQ ID NO: 2 are respectively substituted with Y, G, K, and L.

Another example of the parent lipase is a polypeptide that consists of an amino acid sequence having an identity of at least 80% or 81% with the amino acid sequence of SEQ ID NO: 16 and has lipase activity. Here, the polypeptide consisting of the amino acid sequence of SEQ ID NO: 16 and having lipase activity is lipase PspLip2 (NCBI Accession No. WP_088424928.1) derived from *Pseudomonas* sp. The parent lipase that is a polypeptide consisting of an amino acid sequence having at least the above predetermined identity with the amino acid sequence of SEQ ID NO: 16 and having lipase activity preferably has A at a position corresponding to position 90 of the amino acid sequence of SEQ ID NO: 2 or V at a position corresponding to position 181 of the amino acid sequence of SEQ ID NO: 2, and more preferably has A and V at positions corresponding to positions 90 and 181, respectively, of the amino acid sequence of SEQ ID NO: 2.

When the lipase mutant of the present invention is produced from the parent lipase, in the parent lipase, preferably, the amino acid residue at a position corresponding to position 90 of the amino acid sequence of SEQ ID NO: 2 is substituted with C, the amino acid residue at a position corresponding to position 181 of the amino acid sequence of SEQ ID NO: 2 is substituted with L, or the amino acid residues at positions corresponding to positions 90 and 181 of the amino acid sequence of SEQ ID NO: 2 are respectively substituted with C and L.

When the lipase mutant of the present invention is produced from the parent lipase, more specifically, in the parent lipase, preferably, A at a position corresponding to position 90 of the amino acid sequence of SEQ ID NO: 2 is substituted with C; V at a position corresponding to position 181 of the amino acid sequence of SEQ ID NO: 2 is substituted with L; or A and V at positions corresponding to positions 90 and 181 of the amino acid sequence of SEQ ID NO: 2 are respectively substituted with C and L.

Another example of the parent lipase is a polypeptide that consists of an amino acid sequence having an identity of at least 80% with the amino acid sequence of SEQ ID NO: 34 and has lipase activity. Here, the polypeptide consisting of the amino acid sequence of SEQ ID NO: 16 and having lipase activity is lipase PaLip (NCBI Accession No. WP_062381422.1) derived from *Pseudomonas abietaniphila.* The parent lipase that is a polypeptide consisting of an amino acid sequence having an identity of at least 80% with the amino acid sequence of SEQ ID NO: 16 and having lipase activity preferably has M at a position corresponding to position 29 of the amino acid sequence of SEQ ID NO: 2.

When the lipase mutant of the present invention is produced from the parent lipase, in the parent lipase, the amino acid residue at a position corresponding to position 29 of the amino acid sequence of SEQ ID NO: 2 is substituted with Y.

When the lipase mutant of the present invention is produced from the parent lipase, in the parent lipase, more specifically, M at a position corresponding to position 29 of the amino acid sequence of SEQ ID NO: 2 is substituted with Y.

The present invention also provides a lipase mutant. The lipase mutant of the present invention is a polypeptide that consists of an amino acid sequence in which an amino acid residue at a predetermined position numbered according to SEQ ID NO: 2 in the amino acid sequence of the parent lipase is substituted with a predetermined amino acid residue and has lipase activity.

The lipase mutant of the present invention is specifically any lipase mutant selected from the following (a) to (v) and (aa) to (ae):
(a) a lipase mutant consisting of an amino acid sequence having an identity of at least 82% with the amino acid sequence of SEQ ID NO: 2 and having D at a position corresponding to position 118 of the amino acid sequence of SEQ ID NO: 2;
(b) a lipase mutant consisting of an amino acid sequence having an identity of at least 80% with the amino acid sequence of SEQ ID NO: 4 and having Y at a position corresponding to position 31 of the amino acid sequence of SEQ ID NO: 2;
(c) a lipase mutant consisting of an amino acid sequence having an identity of at least 91% with the amino acid sequence of SEQ ID NO: 4 and having G at a position corresponding to position 32 of the amino acid sequence of SEQ ID NO: 2;
(d) a lipase mutant consisting of an amino acid sequence having an identity of at least 80% with the amino acid sequence of SEQ ID NO: 4 and having C at a position corresponding to position 90 of the amino acid sequence of SEQ ID NO: 2;
(e) a lipase mutant consisting of an amino acid sequence having an identity of at least 94% with the amino acid sequence of SEQ ID NO: 4 and having L at a position corresponding to position 181 of the amino acid sequence of SEQ ID NO: 2;
(f) a lipase mutant consisting of an amino acid sequence having an identity of at least 80% with the amino acid sequence of SEQ ID NO: 6 and having A, T, H, or G at a position corresponding to position 222 of the amino acid sequence of SEQ ID NO: 2;
(g) a lipase mutant consisting of an amino acid sequence having an identity of at least 80% with the amino acid sequence of SEQ ID NO: 8 and having K at a position corresponding to position 39 of the amino acid sequence of SEQ ID NO: 2;
(h) a lipase mutant consisting of an amino acid sequence having an identity of at least 80% with the amino acid sequence of SEQ ID NO: 8 and having T at a position corresponding to position 125 of the amino acid sequence of SEQ ID NO: 2;
(i) a lipase mutant consisting of an amino acid sequence having an identity of at least 80% with the amino acid sequence of SEQ ID NO: 8 and having P at a position corresponding to position 126 of the amino acid sequence of SEQ ID NO: 2;
(j) a lipase mutant consisting of an amino acid sequence having an identity of at least 80% with the amino acid sequence of SEQ ID NO: 8 and having G at a position corresponding to position 293 of the amino acid sequence of SEQ ID NO: 2;
(k) a lipase mutant consisting of an amino acid sequence having an identity of at least 80% with the amino acid sequence of SEQ ID NO: 10 and having Y at a position corresponding to position 29 of the amino acid sequence of SEQ ID NO: 2;
(l) a lipase mutant consisting of an amino acid sequence having an identity of at least 80% with the amino acid sequence of SEQ ID NO: 10 and having Y at a position corresponding to position 31 of the amino acid sequence of SEQ ID NO: 2;
(m) a lipase mutant consisting of an amino acid sequence having an identity of at least 80% with the amino acid sequence of SEQ ID NO: 10 and having G at a position corresponding to position 32 of the amino acid sequence of SEQ ID NO: 2;
(n) a lipase mutant consisting of an amino acid sequence having an identity of at least 80% with the amino acid sequence of SEQ ID NO: 10 and having G at a position corresponding to position 186 of the amino acid sequence of SEQ ID NO: 2;
(o) a lipase mutant consisting of an amino acid sequence having an identity of at least 80% with the amino acid sequence of SEQ ID NO: 12 and having Q at a position corresponding to position 206 of the amino acid sequence of SEQ ID NO: 2;
(p) a lipase mutant consisting of an amino acid sequence having an identity of at least 80% with the amino acid sequence of SEQ ID NO: 14 and having Y at a position corresponding to position 31 of the amino acid sequence of SEQ ID NO: 2;
(q) a lipase mutant consisting of an amino acid sequence having an identity of at least 80% with the amino acid sequence of SEQ ID NO: 14 and having G at a position corresponding to position 32 of the amino acid sequence of SEQ ID NO: 2;
(r) a lipase mutant consisting of an amino acid sequence having an identity of at least 97% with the amino acid sequence of SEQ ID NO: 14 and having K at a position corresponding to position 77 of the amino acid sequence of SEQ ID NO: 2;
(s) a lipase mutant consisting of an amino acid sequence having an identity of at least 83% with the amino acid sequence of SEQ ID NO: 14 and having L at a position corresponding to position 181 of the amino acid sequence of SEQ ID NO: 2;
(t) a lipase mutant consisting of an amino acid sequence having an identity of at least 80% with the amino acid sequence of SEQ ID NO: 16 and having C at a position corresponding to position 90 of the amino acid sequence of SEQ ID NO: 2;
(u) a lipase mutant consisting of an amino acid sequence having an identity of at least 81% with the amino acid sequence of SEQ ID NO: 16 and having L at a position corresponding to position 181 of the amino acid sequence of SEQ ID NO: 2;
(v) a lipase mutant consisting of an amino acid sequence having an identity of at least 80% with the amino acid sequence of SEQ ID NO: 34 and having Y at a position corresponding to position 29 of the amino acid sequence of SEQ ID NO: 2;
(aa) a lipase mutant consisting of an amino acid sequence having an identity of at least 80% with the amino acid sequence of SEQ ID NO: 4 and having at least two amino acid residues selected from Y at a position corresponding to position 31, G at a position corresponding to position 32, C at a position corresponding to position 90, and L at a position corresponding to position 181 of the amino acid sequence of SEQ ID NO: 2;
(ab) a lipase mutant consisting of an amino acid sequence having an identity of at least 80% with the amino acid sequence of SEQ ID NO: 8 and having at least two amino acid residues selected from K at a position corresponding to position 39, T at a position corresponding to position 125, P at a position corresponding to position 126, and G at a position corresponding to position 293 of the amino acid sequence of SEQ ID NO: 2;
(ac) a lipase mutant consisting of an amino acid sequence having an identity of at least 80% with the amino acid sequence of SEQ ID NO: 10 and having at least two amino acid residues selected from Y at a position corresponding to position 29, Y at a position corresponding to position 31, G at a position corresponding to position 32, and G at a position corresponding to position 186 of the amino acid sequence of SEQ ID NO: 2;
(ad) a lipase mutant consisting of an amino acid sequence having an identity of at least 83% with the amino acid sequence of SEQ ID NO: 14 and having at least two amino acid residues selected from Y at a position corresponding to position 31, G at a position corresponding to position 32, K at a position corresponding to position 77, and L at a position corresponding to position 181 of the amino acid sequence of SEQ ID NO: 2; and
(ae) a lipase mutant consisting of an amino acid sequence having an identity of at least 80% with the amino acid sequence of SEQ ID NO: 16 and having C at a position corresponding to position 90 and L at a position corresponding to position 181 of the amino acid sequence of SEQ ID NO: 2.

Among the lipase mutants (a) to (v), preferable lipase mutants are lipase mutants (a), (c) to (g), (i) to (k), (n), (o), (r), and (t) to (v).

The lipase mutant (aa) is preferably any of the following lipase mutants (aa-1) to (aa-4), the lipase mutant (ab) is preferably any of the following lipase mutants (ab-1) to (ab-6), the lipase mutant (ac) is preferably any of the following lipase mutants (ac-1) to (ac-2), and the lipase mutant (ad) is preferably any of the following lipase mutants (ad-1) to (ad-4):
(aa-1) a lipase mutant that consists of an amino acid sequence having an identity of at least 80% with the amino acid sequence of SEQ ID NO: 4 and has Y at a position corresponding to position 31 and G at a position corresponding to position 32 of the amino acid sequence of SEQ ID NO: 2;
(aa-2) a lipase mutant that consists of an amino acid sequence having an identity of at least 80% with the amino acid sequence of SEQ ID NO: 4 and has C at a position corresponding to position 90 and L at a position corresponding to position 181 of the amino acid sequence of SEQ ID NO: 2;
(aa-3) a lipase mutant that consists of an amino acid sequence having an identity of at least 80% with the amino acid sequence of SEQ ID NO: 4 and has G at a position corresponding to position 32 and C at a position corresponding to position 90 of the amino acid sequence of SEQ ID NO: 2;
(aa-4) a lipase mutant that consists of an amino acid sequence having an identity of at least 80% with the amino acid sequence of SEQ ID NO: 4 and has Y at a position corresponding to position 31, G at a position corresponding to position 32, C at a position corresponding to position 90, and L at a position corresponding to position 181 of the amino acid sequence of SEQ ID NO: 2;
(ab-1) a lipase mutant that consists of an amino acid sequence having an identity of at least 80% with the amino acid sequence of SEQ ID NO: 8 and has T at a position corresponding to position 125 and P at a position corresponding to position 126 of the amino acid sequence of SEQ ID NO: 2;
(ab-2) a lipase mutant that consists of an amino acid sequence having an identity of at least 80% with the amino acid sequence of SEQ ID NO: 8 and has K at a position corresponding to position 39 and G at a position corresponding to position 293 of the amino acid sequence of SEQ ID NO: 2;
(ab-3) a lipase mutant that consists of an amino acid sequence having an identity of at least 80% with the amino acid sequence of SEQ ID NO: 8 and has T at a position corresponding to position 125, P at a position corresponding to position 126, and G at a position corresponding to position 293 of the amino acid sequence of SEQ ID NO: 2;
(ab-4) a lipase mutant that consists of an amino acid sequence having an identity of at least 80% with the amino acid sequence of SEQ ID NO: 8 and has K at a position corresponding to position 39, T at a position corresponding to position 125, and P at a position corresponding to position 126 of the amino acid sequence of SEQ ID NO: 2;
(ab-5) a lipase mutant that consists of an amino acid sequence having an identity of at least 80% with the amino acid sequence of SEQ ID NO: 8 and has K at a position corresponding to position 39, P at a position corresponding to position 126, and G at a position corresponding to position 293 of the amino acid sequence of SEQ ID NO: 2;
(ab-6) a lipase mutant that consists of an amino acid sequence having an identity of at least 80% with the amino acid sequence of SEQ ID NO: 8 and has K at a position corresponding to position 39, T at a position corresponding to position 125, P at a position corresponding to position 126, and G at a position corresponding to position 293 of the amino acid sequence of SEQ ID NO: 2;
(ac-1) a lipase mutant that consists of an amino acid sequence having an identity of at least 80% with the amino acid sequence of SEQ ID NO: 10 and has Y at a position corresponding to position 29 and G at a position corresponding to position 186 of the amino acid sequence of SEQ ID NO: 2;
(ac-2) a lipase mutant that consists of an amino acid sequence having an identity of at least 80% with the amino acid sequence of SEQ ID NO: 10 and has Y at a position corresponding to position 29, Y at a position corresponding to position 31, G at a position corresponding to position 32, and G at a position corresponding to position 186 of the amino acid sequence of SEQ ID NO: 2;
(ad-1) a lipase mutant that consists of an amino acid sequence having an identity of at least 83% with the amino acid sequence of SEQ ID NO: 14 and has Y at a position corresponding to position 31 and G at a position corresponding to position 32 of the amino acid sequence of SEQ ID NO: 2;
(ad-2) a lipase mutant that consists of an amino acid sequence having an identity of at least 83% with the amino acid sequence of SEQ ID NO: 14 and has K at a position corresponding to position 77 and L at a position corresponding to position 181 of the amino acid sequence of SEQ ID NO: 2;
(ad-3) a lipase mutant that consists of an amino acid sequence having an identity of at least 83% with the amino acid sequence of SEQ ID NO: 14 and has Y at a position corresponding to position 31, G at a position corresponding to position 32, and L at a position corresponding to position 181 of the amino acid sequence of SEQ ID NO: 2; and
(ad-4) a lipase mutant that consists of an amino acid sequence having an identity of at least 83% with the amino acid sequence of SEQ ID NO: 14 and has Y at a position corresponding to position 31, G at a position corresponding to position 32, K at a position corresponding to position 77, and L at a position corresponding to position 181 of the amino acid sequence of SEQ ID NO: 2.

The substitution of an amino acid residue at the above predetermined position is substitution for improving the heterologous expression of a lipase. Accordingly, the lipase mutant of the present invention has been improved in the expression in different species compared to a parent lipase, i.e., a lipase before substitution of an amino acid residue at a predetermined position.

In the lipase mutant of the present invention, a substitution site (mutation position) of an amino acid residue is indicated by numbering according to SEQ ID NO: 2. In Table 1 below, regarding each parent lipase, a substitution site of an amino acid residue is indicated by numbering according to the amino acid sequence of each parent lipase and numbering according to the amino acid sequence of SEQ ID NO: 2.

**[Table 1]**

| SEQ ID NO: | Name | Numbering according to each parent enzyme | | | | Numbering according to SEQ ID NO: 2 | | | |
|---|---|---|---|---|---|---|---|---|---|
| 2 | SspLip | E118D | | | | E118D | | | |
| 4 | PfLip | F30Y | E31G | A89C | V180L | F31Y | E32G | A90C | V181L |
| 6 | EtLip | I222A/T/H/G | | | | I222A/T/H/G | | | |
| 8 | YeLip | V38K | V124T | E125P | S292G | V39K | V125T | E126P | S293G |
| 10 | AnLip | C24Y | F26Y | A27G | R181G | C29Y | F31Y | A32G | R186G |
| 12 | YfLip | G206Q | | | | G206Q | | | |
| 14 | PspLip | F30Y | D31G | R76K | M179L | F31Y | D32G | R77K | M181L |
| 16 | PspLip2 | A89C | V180L | | | A90C | V181L | | |
| 34 | PaLip | M28Y | | | | M29Y | | | |

As shown in Table 1, substitution of the amino acid residue at a position corresponding to position 29 of the amino acid sequence of SEQ ID NO: 2 with Y is common substitution when the parent lipase is a polypeptide consisting of an amino acid sequence having at least the above predetermined identity with SEQ ID NO: 10 or 34 and having lipase activity. Substitution of the amino acid residue at a position corresponding to position 31 of the amino acid sequence of SEQ ID NO: 2 with Y is common substitution when the parent lipase is a polypeptide consisting of an amino acid sequence having at least the above predetermined identity with SEQ ID NO: 4, 10, or 14 and having lipase activity. Substitution of the amino acid residue at a position corresponding to position 32 of the amino acid sequence of SEQ ID NO: 2 with G is common substitution when the parent lipase is a polypeptide consisting of an amino acid sequence having at least the above predetermined identity with SEQ ID NO: 4, 10, or 14 and having lipase activity. Substitution of an amino acid residue at a position corresponding to position 90 of the amino acid sequence of SEQ ID NO: 2 with C is common substitution when the parent lipase is a polypeptide consisting of an amino acid sequence having at least the above predetermined identity with SEQ ID NO: 4 or 16 and having lipase activity. Substitution of an amino acid residue at a position corresponding to position 181 of the amino acid sequence of SEQ ID NO: 2 with L is common substitution when the parent lipase is a polypeptide consisting of an amino acid sequence having at least the above predetermined identity with SEQ ID NO: 4, 14, or 16 and having lipase activity.

The lipase mutant of the present invention may have a mutation (for example, deletion, substitution, addition, or insertion) at any position of the parent lipase, in addition to the mutations at the above predetermined positions, as long as the heterologous expression is not prevented. The mutation may be naturally generated or may be artificially introduced.

### <2. Polynucleotide encoding lipase mutant of the present invention>

The lipase mutant of the present invention can be produced using various mutagenesis techniques known in the art. For example, the lipase mutant can be produced by mutating a polynucleotide encoding the amino acid residue to be substituted in a parent lipase gene (reference lipase gene) encoding the reference amino acid sequence into a polynucleotide encoding an amino acid residue after substitution, and further expressing the mutant from the mutant gene.

The polynucleotide encoding the lipase mutant of the present invention can be in the form of single-stranded or double-stranded DNA, RNA, or artificial nucleic acid or can be cDNA or chemically synthesized DNA containing no introns.

In the present invention, as the method for mutating an amino acid residue of a parent lipase, various mutagenesis techniques known in the art can be used. For example, a polynucleotide encoding the lipase mutant of the present invention can be obtained by mutating a nucleotide sequence encoding an amino acid residue to be mutated into a nucleotide sequence encoding the amino acid residue after mutation in a polynucleotide encoding an amino acid sequence of a parent lipase (hereinafter, also referred to as parent gene).

Basically, introduction of a desired mutation into a parent gene can be performed using various site-directed mutagenesis methods known to those skilled in the art. The site-directed mutagenesis can be performed by an arbitrary method, such as an inverse PCR method or an annealing method. Commercially available site-directed mutagenesis kits (for example, QuickChange II Site-Directed Mutagenesis Kit and QuickChange Multi Site-Directed Mutagenesis Kit of Stratagene) can also be used.

Site-directed mutagenesis into a parent gene can be most generally performed using a mutation primer including a nucleotide mutation to be introduced. The mutation primer may be designed so as to anneal with a region including a nucleotide sequence that encodes an amino acid residue to be mutated in a parent gene and to include a nucleotide sequence having a nucleotide sequence (codon) that encodes an amino acid residue after mutation instead of the nucleotide sequence (codon) that encodes the amino acid residue to be mutated. Nucleotide sequences (codons) that encode amino acid residues before and after mutation can be appropriately recognized and selected by those skilled in the art based on ordinary textbooks and the like. Alternatively, as site-directed mutagenesis, a method of linking DNA fragments into one by SOE (splicing by overlap extension)-PCR (Gene, 1989, 77(1): 61-68), wherein the DNA fragments are separately obtained by amplifying both the upstream and downstream sides of a mutation site with two complementary primers including the nucleotide mutation to be introduced, can be used.

A template DNA including a parent gene can be prepared from a microorganism that produces the above-described lipase by extracting a genomic DNA by a usual method or extracting RNA and synthesizing a cDNA therefrom by reverse transcription. Alternatively, a corresponding nucleotide sequence chemically synthesized based on the amino acid sequence of a parent lipase may be used as a template DNA. DNA sequences including nucleotide sequences encoding lipases respectively consisting of amino acid sequences of SEQ ID NOs: 2, 4, 6, 8, 10, 12, 14, 16, and 34 are shown respectively by SEQ ID NOs: 1, 3, 5, 7, 9, 11, 13, 15, and 33.

A mutation primer can be produced by a known oligonucleotide synthesis method such as a phosphoramidite method (Nucleic Acids R4esearch, 1989, 17: 7059-7071). Such primer synthesis can also be carried out using, for example, a commercially available oligonucleotide synthesis apparatus (for example, manufactured by ABI Inc.). A polynucleotide encoding a lipase mutant with a desired mutation of the present invention can be obtained using a primer set including the mutation primer and performing site-directed mutagenesis as described above using a parent gene as a template DNA.

The polynucleotide encoding the lipase mutant of the present invention can include a single-stranded or double-stranded DNA, cDNA, RNA, or another artificial nucleic acid. The DNA, cDNA, and RNA may be chemically synthesized. The polynucleotide may include a nucleotide sequence of an untranslated region (UTR), in addition to an open reading frame (ORF). The polynucleotide may be codon-optimized in accordance with the type of a transformant for producing the mutant polypeptide of the present invention. Information on codons to be used by various organisms is available from the Codon Usage Database ([www.kazusa.or.jp/codon/]).

### <3. Vector or DNA fragment>

The obtained polynucleotide encoding the lipase mutant of the present invention can be incorporated into a vector. The type of the vector containing the polynucleotide is not particularly limited and may be any vector such as a plasmid, phage, phagemid, cosmid, virus, YAC vector, and shuttle vector. The vector is, but not limited to, preferably, a vector capable of being amplified in bacteria, preferably, in *Bacillus* bacteria (for example, *Bacillus subtilis* or its mutant strain), more preferably, an expression vector capable of inducing expression of a transgene in *Bacillus* bacteria. Among them, a shuttle vector, which is a vector capable of replicating in any of *Bacillus* bacteria and other organisms, can be suitably used for recombinant production of the lipase mutant of the present invention. Preferable examples of the vector include, but not limited to, shuttle vectors, such as pHA3040SP64, pHSP64R, and pASP64 (Japanese Patent No. 3492935), pHY300PLK (expression vector capable of transforming both *Escherichia coli* and *Bacillus subtilis*; Jpn J. Genet., 1985, 60: 235-243), and pAC3 (Nucleic Acids Res., 1988, 16: 8732); and plasmid vectors that can be used for transformation of *Bacillus* bacteria, such as pUB110 (J. Bacteriol., 1978, 134: 318-329) and pTA10607 (Plasmid, 1987, 18: 8-15). In addition, plasmid vectors derived from *Escherichia coli* (e.g., pET22b(+), pBR322, pBR325, pUC57, pUC118, pUC119, pUC18, pUC19, and pBluescript) can also be used.

The above vectors can include a DNA replication initiation region or a DNA region including replication origin. Alternatively, in the above vectors, a control sequence may be operably linked to the upstream of a polynucleotide encoding the lipase mutant of the present invention (i.e., lipase mutant gene), wherein the control sequence is a promoter region for initiating transcription of the gene, terminator region, secretory signal region for extracellularly secreting the expressed protein, or the like.

The type of the control sequence, such as the promoter region, terminator region, and secretory signal region, is not particularly limited, and a promoter or secretory signal sequence that are usually used can be appropriately selected and used depending on the host into which the vector is introduced. For example, suitable examples of the control sequence capable of being incorporated into a vector include a promoter of the cellulase gene of *Bacllus* sp. KSM-S237 strain and a secretary signal sequence.

Alternatively, a marker gene (e.g., a gene resistant to drugs, such as ampicillin, neomycin, kanamycin, and chloramphenicol) for selecting a host into which the vector has been appropriately introduced may be further introduced into the vector. Alternatively, when an auxotrophic strain is used as the host, a gene encoding an enzyme that synthesizes a required nutrient may be incorporated into a vector as a marker gene. Alternatively, when a selection medium that requires a specific metabolism for growth is used, a gene related to the metabolism may be incorporated into a vector as a marker gene. Examples of such a metabolism-related gene include an acetamidase gene for using acetamide as a nitrogen source.

The polypeptide encoding the lipase mutant of the present invention may be linked to a control sequence and a marker gene by a method known in the art such as an SOE (splicing by overlap extension)-PCR method (Gene, 1989, 77: 61-68). The procedure of introducing the linked fragments into a vector is well known in the art.

### <4. Transformed cell>

A transformed cell of the present invention can be obtained by introducing a vector including a polynucleotide encoding the lipase mutant of the present invention into a host or by introducing a DNA fragment including a polynucleotide encoding the lipase mutant of the present invention into the genome of a host.

Examples of the host cell include microorganisms such as bacteria and fungi. Examples of the bacteria include *Escherichia coli* and bacteria belonging to *Staphylococcus*, *Enterococcus*, *Listeria*, and *Bacillus,* and among them, preferred are *Escherichia coli* and *Bacillus* bacteria, more preferred are *Bacillus* bacteria, and further preferred are *Bacillus subtilis* (e.g., *Bacillus subtilis* Marburg No. 168 strain and its mutant strain). Examples of the *Bacillus subtilis* mutant strain include nine-protease-deficient strain KA8AX described in J. Biosci. Bioeng., 2007, 104(2): 135-143 and D8PA strain that is an eight-protease-deficient strain with improved protein folding efficiency described in Biotechnol. Lett., 2011, 33(9): 1847-1852. Examples of the fungus include *Trichoderma*, *Aspergillus*, and *Rhizopus.*

As the method for introducing a vector into a host, a method that usually used in the art, such as a protoplast method and an electroporation method, can be used. A desired transformant into which a vector has been introduced can be obtained by selecting a strain in which the introduction has been appropriately performed by using expression of a marker gene, auxotrophy, or the like as an indicator.

Alternatively, a fragment in which a polypeptide encoding the lipase mutant of the present invention, a control sequence, and a marker gene are linked can also be directly introduced into the genome of a host. For example, a DNA fragment in which a sequence complementary to the genome of a host has been added to both ends of the linked fragment is constructed by an SOE-PCR method or the like and is introduced into a host to cause homologous recombination between the host genome and the DNA fragment. Consequently, the polynucleotide encoding the lipase mutant of the present invention is introduced into the genome of the host.

When the-thus obtained transformant into which a polynucleotide encoding the lipase mutant of the present invention or a vector including it has been introduced is cultured in an appropriate medium, a gene encoding the protein on the vector is expressed to generate the lipase mutant of the present invention. The medium that is used for culturing the transformant can be appropriately selected depending on the type of the microorganism of the transformant by those skilled in the art.

Alternatively, the lipase mutant of the present invention may be expressed from a polynucleotide encoding the lipase mutant of the present invention or its transcription product using a cell-free translation system. The "cell-free translation system" is an in vitro transcription-translation system or in vitro translation system constructed by adding a reagent, such as amino acid, necessary for translation of a protein to a suspension obtained by mechanically disrupting the cells as the host.

The lipase mutant of the present invention generated by the culture or cell-free translation system can be isolated or purified by using general methods that are used in protein purification, such as centrifugation, ammonium sulfate precipitation, gel chromatography, ion exchange chromatography, and affinity chromatography, alone or in appropriate combination. The protein collected from the culture may be further purified by a known method.

### <5. Method for improving heterologous expression of lipase>

The thus-obtained lipase mutant of the present invention is improved in the heterologous expression compared to the parent lipase.

"Improvement in heterologous expression" means that the expression level when expressed in an organism species different from that from which the parent lipase is derived is increased than the expression level of the parent lipase. Here, heterogeneity is not particularly limited as long as the organism species is different from that from which the parent lipase is derived, but is preferably a microorganism that is broadly used in protein expression systems, more preferably *Escherichia coli* or a *Bacillus* bacterium, further preferably a *Bacillus* bacterium, and further preferably *Bacillus subtilis.* The expression level of the lipase mutant of the present invention may be measured by a method for measuring expression level of a protein known to those skilled in the art or may be measured using the lipase activity of the lipase mutant as an indicator. The heterologous expression of the lipase mutant of the present invention (lipase mutant expression level in different species) can be preferably 130% or more and more preferably 150% or more relative to that of the parent lipase.

Accordingly, in another aspect, the present invention provides a method for improving the heterologous expression of a lipase. The method of the present invention includes substitution of an amino acid residue at a predetermined position numbered according to SEQ ID NO: 2 with a predetermined amino acid residue in the parent lipase. The details of the method are similar to those of the above-described method for producing the lipase mutant of the present invention.

### <6. Application of lipase mutant>

Lipases catalyze various reactions, such as ester synthesis reaction, transesterification reaction, and acidolysis, in addition to ester hydrolysis reaction. By utilizing this reactivity, lipases are used in various industrial applications such as cleansing, optical resolution, biofuel manufacturing, fat/oil processing, wastewater treatment, pulp manufacturing, and leather manufacturing. The lipase mutant of the present invention has improved heterologous expression compared to the parent lipase, thus can be produced in a large quantity industrially advantageously by heterologous expression and can be suitably used in such industrial applications. The lipase mutant sequence group of the present invention is diverse in the sequence and has various different characteristics and therefore can be used in searching for a suitable lipase depending on the substrate or application. Accordingly, the lipase mutant of the present invention can be a detergent, an optical resolution agent, an ester synthesis catalyst, a fat/oil processing catalyst, a wastewater treatment agent, a pulp treatment agent, a leather treatment agent, and so on and can be preferably a detergent. Examples of the detergent include a laundry detergent, a dishwashing detergent, a bleaching agent, a hard surface cleaning detergent, a drain detergent, a denture detergent, and a disinfectant detergent for medical instruments.

The above-mentioned agents may be the lipase mutant of the present invention alone or may be an enzyme composition containing the lipase mutant. The enzyme composition may be a solid composition such as a powder or may be a liquid composition.

The enzyme composition containing the lipase mutant of the present invention can be appropriately blended with an arbitrary additive, such as an inert carrier, a pH adjuster, a dispersant, a buffer, and a preservative, in addition to the lipase mutant. The lipase mutants may be included in the enzyme composition singly or in combination of two or more kinds.

The content of the lipase mutant of the present invention in the enzyme composition of the present invention is not particularly limited as long as the lipase mutant shows the activity in such an amount, and is, for example, preferably 0.1 mg or more, more preferably 1 mg or more, and more preferably 5 mg or more and preferably 50 000 mg or less, more preferably 5 000 mg or less, and more preferably 500 mg or less per kg of the enzyme composition. The content is preferably from 0.1 to 50 000 mg, more preferably from 1 to 5 000 mg, and more preferably from 5 to 500 mg.

Regarding the above-described embodiments, in the present invention, the following aspects are further disclosed.
<1> A lipase mutant selected from the following (a) to (v):
   (a) a lipase mutant consisting of an amino acid sequence having an identity of at least 82% with the amino acid sequence of SEQ ID NO: 2 and having D at a position corresponding to position 118 of the amino acid sequence of SEQ ID NO: 2;
   (b) a lipase mutant consisting of an amino acid sequence having an identity of at least 80% with the amino acid sequence of SEQ ID NO: 4 and having Y at a position corresponding to position 31 of the amino acid sequence of SEQ ID NO: 2;
   (c) a lipase mutant consisting of an amino acid sequence having an identity of at least 91% with the amino acid sequence of SEQ ID NO: 4 and having G at a position corresponding to position 32 of the amino acid sequence of SEQ ID NO: 2;
   (d) a lipase mutant consisting of an amino acid sequence having an identity of at least 80% with the amino acid sequence of SEQ ID NO: 4 and having C at a position corresponding to position 90 of the amino acid sequence of SEQ ID NO: 2;
   (e) a lipase mutant consisting of an amino acid sequence having an identity of at least 94% with the amino acid sequence of SEQ ID NO: 4 and having L at a position corresponding to position 181 of the amino acid sequence of SEQ ID NO: 2;
   (f) a lipase mutant consisting of an amino acid sequence having an identity of at least 80% with the amino acid sequence of SEQ ID NO: 6 and having A, T, H, or G at a position corresponding to position 222 of the amino acid sequence of SEQ ID NO: 2;
   (g) a lipase mutant consisting of an amino acid sequence having an identity of at least 80% with the amino acid sequence of SEQ ID NO: 8 and having K at a position corresponding to position 39 of the amino acid sequence of SEQ ID NO: 2;
   (h) a lipase mutant consisting of an amino acid sequence having an identity of at least 80% with the amino acid sequence of SEQ ID NO: 8 and having T at a position corresponding to position 125 of the amino acid sequence of SEQ ID NO: 2;
   (i) a lipase mutant consisting of an amino acid sequence having an identity of at least 80% with the amino acid sequence of SEQ ID NO: 8 and having P at a position corresponding to position 126 of the amino acid sequence of SEQ ID NO: 2;
   (j) a lipase mutant consisting of an amino acid sequence having an identity of at least 80% with the amino acid sequence of SEQ ID NO: 8 and having G at a position corresponding to position 293 of the amino acid sequence of SEQ ID NO: 2;
   (k) a lipase mutant consisting of an amino acid sequence having an identity of at least 80% with the amino acid sequence of SEQ ID NO: 10 and having Y at a position corresponding to position 29 of the amino acid sequence of SEQ ID NO: 2;
   (l) a lipase mutant consisting of an amino acid sequence having an identity of at least 80% with the amino acid sequence of SEQ ID NO: 10 and having Y at a position corresponding to position 31 of the amino acid sequence of SEQ ID NO: 2;
   (m) a lipase mutant consisting of an amino acid sequence having an identity of at least 80% with the amino acid sequence of SEQ ID NO: 10 and having G at a position corresponding to position 32 of the amino acid sequence of SEQ ID NO: 2;
   (n) a lipase mutant consisting of an amino acid sequence having an identity of at least 80% with the amino acid sequence of SEQ ID NO: 10 and having G at a position corresponding to position 186 of the amino acid sequence of SEQ ID NO: 2;
   (o) a lipase mutant consisting of an amino acid sequence having an identity of at least 80% with the amino acid sequence of SEQ ID NO: 12 and having Q at a position corresponding to position 206 of the amino acid sequence of SEQ ID NO: 2;
   (p) a lipase mutant consisting of an amino acid sequence having an identity of at least 80% with the amino acid sequence of SEQ ID NO: 14 and having Y at a position corresponding to position 31 of the amino acid sequence of SEQ ID NO: 2;
   (q) a lipase mutant consisting of an amino acid sequence having an identity of at least 80% with the amino acid sequence of SEQ ID NO: 14 and having G at a position corresponding to position 32 of the amino acid sequence of SEQ ID NO: 2;
   (r) a lipase mutant consisting of an amino acid sequence having an identity of at least 97% with the amino acid sequence of SEQ ID NO: 14 and having K at a position corresponding to position 77 of the amino acid sequence of SEQ ID NO: 2;
   (s) a lipase mutant consisting of an amino acid sequence having an identity of at least 83% with the amino acid sequence of SEQ ID NO: 14 and having L at a position corresponding to position 181 of the amino acid sequence of SEQ ID NO: 2;
   (t) a lipase mutant consisting of an amino acid sequence having an identity of at least 80% with the amino acid sequence of SEQ ID NO: 16 and having C at a position corresponding to position 90 of the amino acid sequence of SEQ ID NO: 2;
   (u) a lipase mutant consisting of an amino acid sequence having an identity of at least 81% with the amino acid sequence of SEQ ID NO: 16 and having L at a position corresponding to position 181 of the amino acid sequence of SEQ ID NO: 2; and
   (v) a lipase mutant consisting of an amino acid sequence having an identity of at least 80% with the amino acid sequence of SEQ ID NO: 34 and having Y at a position corresponding to position 29 of the amino acid sequence of SEQ ID NO: 2.
<2> A lipase mutant selected from the following (aa) to (ae) :
   (aa) a lipase mutant consisting of an amino acid sequence having an identity of at least 80% with the amino acid sequence of SEQ ID NO: 4 and having at least two amino acid residues selected from Y at a position corresponding to position 31, G at a position corresponding to position 32, C at a position corresponding to position 90, and L at a position corresponding to position 181 of the amino acid sequence of SEQ ID NO: 2;
   (ab) a lipase mutant consisting of an amino acid sequence having an identity of at least 80% with the amino acid sequence of SEQ ID NO: 8 and having at least two amino acid residues selected from K at a position corresponding to position 39, T at a position corresponding to position 125, P at a position corresponding to position 126, and G at a position corresponding to position 293 of the amino acid sequence of SEQ ID NO: 2;
   (ac) a lipase mutant consisting of an amino acid sequence having an identity of at least 80% with the amino acid sequence of SEQ ID NO: 10 and having at least two amino acid residues selected from Y at a position corresponding to position 29, Y at a position corresponding to position 31, G at a position corresponding to position 32, and G at a position corresponding to position 186 of the amino acid sequence of SEQ ID NO: 2;
   (ad) a lipase mutant consisting of an amino acid sequence having an identity of at least 83% with the amino acid sequence of SEQ ID NO: 14 and having at least two amino acid residues selected from Y at a position corresponding to position 31, G at a position corresponding to position 32, K at a position corresponding to position 77, and L at a position corresponding to position 181 of the amino acid sequence of SEQ ID NO: 2; and
   (ae) a lipase mutant consisting of an amino acid sequence having an identity of at least 80% with the amino acid sequence of SEQ ID NO: 16 and having C at a position corresponding to position 90 and L at a position corresponding to position 181 of the amino acid sequence of SEQ ID NO: 2.
<3> The lipase mutant according to <1>, wherein the lipase mutant is any lipase mutant selected from the (a), (c) to (g), (i) to (k), (n), (o), (r), and (t) to (v).
<4> The lipase mutant according to <2>, wherein the lipase mutant is any lipase mutant selected from the following (aa-1) to (ae):
   (aa-1) a lipase mutant that consists of an amino acid sequence having an identity of at least 80% with the amino acid sequence of SEQ ID NO: 4 and has Y at a position corresponding to position 31 and G at a position corresponding to position 32 of the amino acid sequence of SEQ ID NO: 2;
   (aa-2) a lipase mutant that consists of an amino acid sequence having an identity of at least 80% with the amino acid sequence of SEQ ID NO: 4 and has C at a position corresponding to position 90 and L at a position corresponding to position 181 of the amino acid sequence of SEQ ID NO: 2;
   (aa-3) a lipase mutant that consists of an amino acid sequence having an identity of at least 80% with the amino acid sequence of SEQ ID NO: 4 and has G at a position corresponding to position 32 and C at a position corresponding to position 90 of the amino acid sequence of SEQ ID NO: 2;
   (aa-4) a lipase mutant that consists of an amino acid sequence having an identity of at least 80% with the amino acid sequence of SEQ ID NO: 4 and has Y at a position corresponding to position 31, G at a position corresponding to position 32, C at a position corresponding to position 90, and L at a position corresponding to position 181 of the amino acid sequence of SEQ ID NO: 2;
   (ab-1) a lipase mutant that consists of an amino acid sequence having an identity of at least 80% with the amino acid sequence of SEQ ID NO: 8 and has T at a position corresponding to position 125 and P at a position corresponding to position 126 of the amino acid sequence of SEQ ID NO: 2;
   (ab-2) a lipase mutant that consists of an amino acid sequence having an identity of at least 80% with the amino acid sequence of SEQ ID NO: 8 and has K at a position corresponding to position 39 and G at a position corresponding to position 293 of the amino acid sequence of SEQ ID NO: 2;
   (ab-3) a lipase mutant that consists of an amino acid sequence having an identity of at least 80% with the amino acid sequence of SEQ ID NO: 8 and has T at a position corresponding to position 125, P at a position corresponding to position 126, and G at a position corresponding to position 293 of the amino acid sequence of SEQ ID NO: 2;
   (ab-4) a lipase mutant that consists of an amino acid sequence having an identity of at least 80% with the amino acid sequence of SEQ ID NO: 8 and has K at a position corresponding to position 39, T at a position corresponding to position 125, and P at a position corresponding to position 126 of the amino acid sequence of SEQ ID NO: 2;
   (ab-5) a lipase mutant that consists of an amino acid sequence having an identity of at least 80% with the amino acid sequence of SEQ ID NO: 8 and has K at a position corresponding to position 39, P at a position corresponding to position 126, and G at a position corresponding to position 293 of the amino acid sequence of SEQ ID NO: 2;
   (ab-6) a lipase mutant that consists of an amino acid sequence having an identity of at least 80% with the amino acid sequence of SEQ ID NO: 8 and has K at a position corresponding to position 39, T at a position corresponding to position 125, P at a position corresponding to position 126, and G at a position corresponding to position 293 of the amino acid sequence of SEQ ID NO: 2;
   (ac-1) a lipase mutant that consists of an amino acid sequence having an identity of at least 80% with the amino acid sequence of SEQ ID NO: 10 and has Y at a position corresponding to position 29 and G at a position corresponding to position 186 of the amino acid sequence of SEQ ID NO: 2;
   (ac-2) a lipase mutant that consists of an amino acid sequence having an identity of at least 80% with the amino acid sequence of SEQ ID NO: 10 and has Y at a position corresponding to position 29, Y at a position corresponding to position 31, G at a position corresponding to position 32, and G at a position corresponding to position 186 of the amino acid sequence of SEQ ID NO: 2;
   (ad-1) a lipase mutant that consists of an amino acid sequence having an identity of at least 83% with the amino acid sequence of SEQ ID NO: 14 and has Y at a position corresponding to position 31 and G at a position corresponding to position 32 of the amino acid sequence of SEQ ID NO: 2;
   (ad-2) a lipase mutant that consists of an amino acid sequence having an identity of at least 83% with the amino acid sequence of SEQ ID NO: 14 and has K at a position corresponding to position 77 and L at a position corresponding to position 181 of the amino acid sequence of SEQ ID NO: 2;
   (ad-3) a lipase mutant that consists of an amino acid sequence having an identity of at least 83% with the amino acid sequence of SEQ ID NO: 14 and has Y at a position corresponding to position 31, G at a position corresponding to position 32, and L at a position corresponding to position 181 of the amino acid sequence of SEQ ID NO: 2;
   (ad-4) a lipase mutant that consists of an amino acid sequence having an identity of at least 83% with the amino acid sequence of SEQ ID NO: 14 and has Y at a position corresponding to position 31, G at a position corresponding to position 32, K at a position corresponding to position 77, and L at a position corresponding to position 181 of the amino acid sequence of SEQ ID NO: 2; and
   (ae) a lipase mutant consisting of an amino acid sequence having an identity of at least 80% with the amino acid sequence of SEQ ID NO: 16 and having cysteine at a position corresponding to position 90 and leucine at a position corresponding to position 181 of the amino acid sequence of SEQ ID NO: 2.
<5> An enzyme composition comprising the lipase mutant according to any one of <1> to <4>.
<6> The enzyme composition according to <5>, wherein the enzyme composition is a detergent, an optical resolution agent, an ester synthesis catalyst, a fat/oil processing catalyst, a wastewater treatment agent, a pulp treatment agent, or a leather treatment agent and preferably a detergent.
<7> A polynucleotide encoding the lipase mutant according to any one of <1> to <4>.
<8> A vector or DNA fragment comprising the polynucleotide according to <7>.
<9> A transformed cell comprising the vector or DNA fragment according to <7>.
<10> The transformed cell according to <9>, wherein the transformed cell is a microorganism.
<11> The transformed cell according to <10>, wherein the transformed cell is *Escherichia coli* or a *Bacillus* bacterium, preferably a *Bacillus* bacterium, and more preferably *Bacillus subtilis.*
<12> A method for producing a lipase mutant, comprising a step of culturing the transformed cell according to any one of <9> to <11>.

<13> A method for producing a lipase mutant, comprising a step selected from the following (i) to (xxii):
   (i) a step of substituting an amino acid residue at a position corresponding to position 118 of the amino acid sequence of SEQ ID NO: 2 with D in a polypeptide that consists of an amino acid sequence having an identity of at least 82% with the amino acid sequence of SEQ ID NO: 2 and has lipase activity;
   (ii) a step of substituting an amino acid residue at a position corresponding to position 31 of the amino acid sequence of SEQ ID NO: 2 with Y in a polypeptide that consists of an amino acid sequence having an identity of at least 80% with the amino acid sequence of SEQ ID NO: 4 and has lipase activity;
   (iii) a step of substituting an amino acid residue at a position corresponding to position 32 of the amino acid sequence of SEQ ID NO: 2 with G in a polypeptide that consists of an amino acid sequence having an identity of at least 91% with the amino acid sequence of SEQ ID NO: 4 and has lipase activity;
   (iv) a step of substituting an amino acid residue at a position corresponding to position 90 of the amino acid sequence of SEQ ID NO: 2 with C in a polypeptide that consists of an amino acid sequence having an identity of at least 80% with the amino acid sequence of SEQ ID NO: 4 and has lipase activity;
   (v) a step of substituting an amino acid residue at a position corresponding to position 181 of the amino acid sequence of SEQ ID NO: 2 with L in a polypeptide that consists of an amino acid sequence having an identity of at least 94% with the amino acid sequence of SEQ ID NO: 4 and has lipase activity;
   (vi) a step of substituting an amino acid residue at a position corresponding to position 222 of the amino acid sequence of SEQ ID NO: 2 with A, T, H, or G in a polypeptide that consists of an amino acid sequence having an identity of at least 80% with the amino acid sequence of SEQ ID NO: 6 and has lipase activity;
   (vii) a step of substituting an amino acid residue at a position corresponding to position 39 of the amino acid sequence of SEQ ID NO: 2 with K in a polypeptide that consists of an amino acid sequence having an identity of at least 80% with the amino acid sequence of SEQ ID NO: 8 and has lipase activity;
   (viii) a step of substituting an amino acid residue at a position corresponding to position 125 of the amino acid sequence of SEQ ID NO: 2 with T in a polypeptide that consists of an amino acid sequence having an identity of at least 80% with the amino acid sequence of SEQ ID NO: 8 and has lipase activity;
   (ix) a step of substituting an amino acid residue at a position corresponding to position 126 of the amino acid sequence of SEQ ID NO: 2 with P in a polypeptide that consists of an amino acid sequence having an identity of at least 80% with the amino acid sequence of SEQ ID NO: 8 and has lipase activity;
   (x) a step of substituting an amino acid residue at a position corresponding to position 293 of the amino acid sequence of SEQ ID NO: 2 with G in a polypeptide that consists of an amino acid sequence having an identity of at least 80% with the amino acid sequence of SEQ ID NO: 8 and has lipase activity;
   (xi) a step of substituting an amino acid residue at a position corresponding to position 29 of the amino acid sequence of SEQ ID NO: 2 with Y in a polypeptide that consists of an amino acid sequence having an identity of at least 80% with the amino acid sequence of SEQ ID NO: 10 and has lipase activity;
   (xii) a step of substituting an amino acid residue at a position corresponding to position 31 of the amino acid sequence of SEQ ID NO: 2 with Y in a polypeptide that consists of an amino acid sequence having an identity of at least 80% with the amino acid sequence of SEQ ID NO: 10 and has lipase activity;
   (xiii) a step of substituting an amino acid residue at a position corresponding to position 32 of the amino acid sequence of SEQ ID NO: 2 with G in a polypeptide that consists of an amino acid sequence having an identity of at least 80% with the amino acid sequence of SEQ ID NO: 10 and has lipase activity;
   (xiv) a step of substituting an amino acid residue at a position corresponding to position 186 of the amino acid sequence of SEQ ID NO: 2 with G in a polypeptide that consists of an amino acid sequence having an identity of at least 80% with the amino acid sequence of SEQ ID NO: 10 and has lipase activity;
   (xv) a step of substituting an amino acid residue at a position corresponding to position 206 of the amino acid sequence of SEQ ID NO: 2 with Q in a polypeptide that consists of an amino acid sequence having an identity of at least 80% with the amino acid sequence of SEQ ID NO: 12 and has lipase activity;
   (xvi) a step of substituting an amino acid residue at a position corresponding to position 31 of the amino acid sequence of SEQ ID NO: 2 with Y in a polypeptide that consists of an amino acid sequence having an identity of at least 80% with the amino acid sequence of SEQ ID NO: 14 and has lipase activity;
   (xvii) a step of substituting an amino acid residue at a position corresponding to position 32 of the amino acid sequence of SEQ ID NO: 2 with G in a polypeptide that consists of an amino acid sequence having an identity of at least 80% with the amino acid sequence of SEQ ID NO: 14 and has lipase activity;
   (xviii) a step of substituting an amino acid residue at a position corresponding to position 77 of the amino acid sequence of SEQ ID NO: 2 with K in a polypeptide that consists of an amino acid sequence having an identity of at least 97% with the amino acid sequence of SEQ ID NO: 14 and has lipase activity;
   (xix) a step of substituting an amino acid residue at a position corresponding to position 181 of the amino acid sequence of SEQ ID NO: 2 with L in a polypeptide that consists of an amino acid sequence having an identity of at least 83% with the amino acid sequence of SEQ ID NO: 14 and has lipase activity;
   (xx) a step of substituting an amino acid residue at a position corresponding to position 90 of the amino acid sequence of SEQ ID NO: 2 with C in a polypeptide that consists of an amino acid sequence having an identity of at least 80% with the amino acid sequence of SEQ ID NO: 16 and has lipase activity;
   (xxi) a step of substituting an amino acid residue at a position corresponding to position 181 of the amino acid sequence of SEQ ID NO: 2 with L in a polypeptide that consists of an amino acid sequence having an identity of at least 81% with the amino acid sequence of SEQ ID NO: 16 and has lipase activity; and
   (xxii) a step of substituting an amino acid residue at a position corresponding to position 29 of the amino acid sequence of SEQ ID NO: 2 with Y in a polypeptide that consists of an amino acid sequence having an identity of at least 80% with the amino acid sequence of SEQ ID NO: 34 and has lipase activity.
<14> A method for producing a lipase mutant, comprising a step selected from the following (xxiii) to (xxvii):
   (xxiii) a step of substituting at least two amino acid residues at positions selected from positions corresponding to positions 31, 32, 90, and 181 of the amino acid sequence of SEQ ID NO: 2 with Y at a position corresponding to position 31, G at a position corresponding to position 32, C at a position corresponding to position 90, and L at a position corresponding to position 181 in a polypeptide that consists of an amino acid sequence having an identity of at least 80% with the amino acid sequence of SEQ ID NO: 4 and has lipase activity;
   (xxiv) a step of substituting at least two amino acid residues at positions selected from positions corresponding to positions 39, 125, 126, and 293 of the amino acid sequence of SEQ ID NO: 2 with K at a position corresponding to position 39, T at a position corresponding to position 125, P at a position corresponding to position 126, and G at a position corresponding to position 293 in a polypeptide that consists of an amino acid sequence having an identity of at least 80% with the amino acid sequence of SEQ ID NO: 8 and has lipase activity;
   (xxv) a step of substituting at least two amino acid residues at positions selected from positions corresponding to positions 29, 31, 32, and 186 of the amino acid sequence of SEQ ID NO: 2 with Y at a position corresponding to position 29, Y at a position corresponding to position 31, G at a position corresponding to position 32, and G at a position corresponding to position 186 in a polypeptide that consists of an amino acid sequence having an identity of at least 80% with the amino acid sequence of SEQ ID NO: 10 and has lipase activity;
   (xxvi) a step of substituting at least two amino acid residues at positions selected from positions corresponding to positions 31, 32, 77, and 181 of the amino acid sequence of SEQ ID NO: 2 with Y at a position corresponding to position 31, G at a position corresponding to position 32, K at a position corresponding to position 77, and L at a position corresponding to position 181 in a polypeptide that consists of an amino acid sequence having an identity of at least 83% with the amino acid sequence of SEQ ID NO: 14 and has lipase activity; and
   (xxvii) a step of substituting an amino acid residue at a position corresponding to position 90 of the amino acid sequence of SEQ ID NO: 2 with C and an amino acid residue at a position corresponding to position 181 with L in a polypeptide that consists of an amino acid sequence having an identity of at least 80% with the amino acid sequence of SEQ ID NO: 16 and has lipase activity.
<15> A method for improving heterologous expression of a lipase, comprising a step selected from the above (i) to (xxii).
<16> A method for improving heterologous expression of a lipase, comprising a step selected from the above (xxiii) to (xxvii).
<17> The method according to <13> or <15>, wherein the step is any step selected from the above (i), (iii) to (vii), (ix) to (xi), (xiv), (xv), (xviii), and (xx) to (xxii).
<18> The method according to <14> or <16>, wherein the step is any step selected from the following (xxiii-1) to (xxvii) :
   (xxiii-1) a step of substituting an amino acid residue at a position corresponding to position 31 of the amino acid sequence of SEQ ID NO: 2 with Y and an amino acid residue at a position corresponding to position 32 with G in a polypeptide that consists of an amino acid sequence having an identity of at least 80% with the amino acid sequence of SEQ ID NO: 4 and has lipase activity;
   (xxiii-2) a step of substituting an amino acid residue at a position corresponding to position 90 of the amino acid sequence of SEQ ID NO: 2 with C and an amino acid residue at a position corresponding to position 181 with L in a polypeptide that consists of an amino acid sequence having an identity of at least 80% with the amino acid sequence of SEQ ID NO: 4 and has lipase activity;
   (xxiii-3) a step of substituting an amino acid residue at a position corresponding to position 32 of the amino acid sequence of SEQ ID NO: 2 with G and an amino acid residue at a position corresponding to position 90 with C in a polypeptide that consists of an amino acid sequence having an identity of at least 80% with the amino acid sequence of SEQ ID NO: 4 and has lipase activity;
   (xxiii-4) a step of substituting an amino acid residue at a position corresponding to position 31 of the amino acid sequence of SEQ ID NO: 2 with Y, an amino acid residue at a position corresponding to position 32 with G, an amino acid residue at a position corresponding to position 90 with C and an amino acid residue at a position corresponding to position 181 with L in a polypeptide that consists of an amino acid sequence having an identity of at least 80% with the amino acid sequence of SEQ ID NO: 4 and has lipase activity;
   (xxiv-1) a step of substituting an amino acid residue at a position corresponding to position 125 of the amino acid sequence of SEQ ID NO: 2 with T and an amino acid residue at a position corresponding to position 126 with P in a polypeptide that consists of an amino acid sequence having an identity of at least 80% with the amino acid sequence of SEQ ID NO: 8 and has lipase activity;
   (xxiv-2) a step of substituting an amino acid residue at a position corresponding to position 39 of the amino acid sequence of SEQ ID NO: 2 with K and an amino acid residue at a position corresponding to position 293 with G in a polypeptide that consists of an amino acid sequence having an identity of at least 80% with the amino acid sequence of SEQ ID NO: 8 and has lipase activity;
   (xxiv-3) a step of substituting an amino acid residue at a position corresponding to position 125 of the amino acid sequence of SEQ ID NO: 2 with T, an amino acid residue at a position corresponding to position 126 with P and an amino acid residue at a position corresponding to position 293 with G in a polypeptide that consists of an amino acid sequence having an identity of at least 80% with the amino acid sequence of SEQ ID NO: 8 and has lipase activity;
   (xxiv-4) a step of substituting an amino acid residue at a position corresponding to position 39 of the amino acid sequence of SEQ ID NO: 2 with K, an amino acid residue at a position corresponding to position 125 with T and an amino acid residue at a position corresponding to position 126 with P in a polypeptide that consists of an amino acid sequence having an identity of at least 80% with the amino acid sequence of SEQ ID NO: 8 and has lipase activity;
   (xxiv-5) a step of substituting an amino acid residue at a position corresponding to position 39 of the amino acid sequence of SEQ ID NO: 2 with K, an amino acid residue at a position corresponding to position 126 with P and an amino acid residue at a position corresponding to position 293 with G in a polypeptide that consists of an amino acid sequence having an identity of at least 80% with the amino acid sequence of SEQ ID NO: 8 and has lipase activity;
   (xxiv-6) a step of substituting an amino acid residue at a position corresponding to position 39 of the amino acid sequence of SEQ ID NO: 2 with K, an amino acid residue at a position corresponding to position 125 with T, an amino acid residue at a position corresponding to position 126 with P and an amino acid residue at a position corresponding to position 293 with G in a polypeptide that consists of an amino acid sequence having an identity of at least 80% with the amino acid sequence of SEQ ID NO: 8 and has lipase activity ;
   (xxv-1) a step of substituting an amino acid residue at a position corresponding to position 29 of the amino acid sequence of SEQ ID NO: 2 with Y and an amino acid residue at a position corresponding to position 186 with G in a polypeptide that consists of an amino acid sequence having an identity of at least 80% with the amino acid sequence of SEQ ID NO: 10 and has lipase activity;
   (xxv-2) a step of substituting an amino acid residue at a position corresponding to position 29 of the amino acid sequence of SEQ ID NO: 2 with Y, an amino acid residue at a position corresponding to position 31 with Y, an amino acid residue at a position corresponding to position 32 with G and an amino acid residue at a position corresponding to position 186 with G in a polypeptide that consists of an amino acid sequence having an identity of at least 80% with the amino acid sequence of SEQ ID NO: 10 and has lipase activity ;
   (xxvi-1) a step of substituting an amino acid residue at a position corresponding to position 31 of the amino acid sequence of SEQ ID NO: 2 with Y and an amino acid residue at a position corresponding to position 32 with G in a polypeptide that consists of an amino acid sequence having an identity of at least 83% with the amino acid sequence of SEQ ID NO: 14 and has lipase activity;
   (xxvi-2) a step of substituting an amino acid residue at a position corresponding to position 77 of the amino acid sequence of SEQ ID NO: 2 with K and an amino acid residue at a position corresponding to position 181 with L in a polypeptide that consists of an amino acid sequence having an identity of at least 83% with the amino acid sequence of SEQ ID NO: 14 and has lipase activity;
   (xxvi-3) a step of substituting an amino acid residue at a position corresponding to position 31 of the amino acid sequence of SEQ ID NO: 2 with Y, an amino acid residue at a position corresponding to position 32 with G and an amino acid residue at a position corresponding to position 181 with L in a polypeptide that consists of an amino acid sequence having an identity of at least 83% with the amino acid sequence of SEQ ID NO: 14 and has lipase activity ;
   (xxvi-4) a step of substituting an amino acid residue at a position corresponding to position 31 of the amino acid sequence of SEQ ID NO: 2 with Y, an amino acid residue at a position corresponding to position 32 with G, an amino acid residue at a position corresponding to position 77 with K and an amino acid residue at a position corresponding to position 181 with L in a polypeptide that consists of an amino acid sequence having an identity of at least 83% with the amino acid sequence of SEQ ID NO: 14 and has lipase activity; and
   (xxvii) a step of substituting an amino acid residue at a position corresponding to position 90 of the amino acid sequence of SEQ ID NO: 2 with C and an amino acid residue at a position corresponding to position 181 with L in a polypeptide that consists of an amino acid sequence having an identity of at least 80% with the amino acid sequence of SEQ ID NO: 16 and has lipase activity.
<19> The method according to any one of <13> to <18>, wherein the lipase mutant has improved heterologous expression compared to a parent lipase.

### Examples

The present invention will be described in further detail based on Examples below, but is not limited thereto.

### (1) Construction of lipase expression plasmid

A lipase expression plasmid was constructed using a plasmid for expressing the VHH of SEQ ID NO: 26 including a promoter derived from *Bacillus subtilis* spoVG gene described in WO 2021/153129 as a template and transferring each lipase gene to the full-length ORF including the VHH gene of the plasmid by an In-Fusion reaction. Plasmids pHY-SspLip, pHY-PfLip, pHY-EtLip, pHY-YeLip, pHY-AnLip, pHY-YfLip, pHY-PspLip, pHY-PspLip2, pHY-SaLip, pHY-BbLip, pHY-EspLip, pHY-AspLip, pHY-YeLip2, pHY-YmLip, pHY-EspLip2, pHY-MiLip, and pHY-PaLip were constructed respectively from artificially synthesized lipase genes SspLip, PfLip, EtLip, YeLip, AnLip, YfLip, PspLip, PspLip2, SaLip, BbLip, EspLip, AspLip, YeLip2, YmLip, EspLip2, MiLip, and PaLip (polynucleotides of SEQ ID NOs: 1, 3, 5, 7, 9, 11, 13, 15, 17, 19, 21, 23, 25, 27, 29, 31, and 33 respectively encoding the amino acid sequences of SEQ ID NOs: 2, 4, 6, 8, 10, 12, 14, 16, 18, 20, 22, 24, 26, 28, 30, 32, and 34). Mutagenesis on each lipase was performed by site-directed introduction by PCR using a complementary primer pair (see Nucleic Acids Research, 2004, 32(14): e115). The mutation introduced into each lipase and the mutation position (position relative to the parent enzyme and the position relative to SEQ ID NO: 2) are shown in Table 2. In the following Examples, the mutation position of each lipase is shown by the position relative to the parent enzyme.

**[Table 2]**

| | | Mutation position | | | | | | | |
|---|---|---|---|---|---|---|---|---|---|
| SEQ ID NO: | Name | Numbering according to each parent enzyme | | | | Numbering according to SEQ ID NO: 2 | | | |
| 2 | SspLip | E118D | | | | E118D | | | |
| 4 | PfLip | F30Y | E31G | A89C | V180L | F31Y | E32G | A90C | V181L |
| 6 | EtLip | I222A/T/H/G | | | | I222A/T/H/G | | | |
| 8 | YeLip | V38K | V124T | E125P | S292G | V39K | V125T | E126P | S293G |
| 10 | AnLip | C24Y | F26Y | A27G | R181G | C29Y | F31Y | A32G | R186G |
| 12 | YfLip | G206Q | | | | G206Q | | | |
| 14 | PspLip | F30Y | D31G | R76K | M179L | F31Y | D32G | R77K | M181L |
| 16 | PspLip2 | A89C | V180L | | | A90C | V181L | | |
| 34 | PaLip | M28Y | | | | M29Y | | | |

### (2) Evaluation of expression of wild-type lipase

From a public database, 16 various sequences (SspLip, PfLip, EtLip, YeLip, AnLip, YfLip, PspLip, PspLip2, SaLip, BbLip, EspLip, AspLip, YeLip2, YmLip, EspLip2, MiLip (SEQ ID NOs: 2, 4, 6, 8, 10, 12, 14, 16, 18, 20, 22, 24, 26, 28, 30, and 32 respectively)) belonging to the *Proteus*/*Yersinia* clade were selected, and their heterologous expression was evaluated. The wild-type lipase expression plasmid obtained in (1) or an empty vector pHY300PLK (Takara Bio Inc.) was introduced into *Bacillus subtilis* Dpr9 strain (see Microbial cell factories 20.1 (2021): 1-13) by a protoplast method, the strain was cultured in a 2× L-maltose medium (2% tryptone, 1% yeast extract, 1% NaCl, 7.5% maltose, 7.5 ppm manganese sulfate pentahydrate, 0.04% calcium chloride dihydrate, and 10 ppm tetracycline; % is (w/v)%) at 30°C for 3 days, and the culture supernatant was then collected by centrifugation. The culture supernatant was mixed with an equal amount of 2× Laemmli Sample buffer (Bio-Rad Laboratories, Inc.) including 100 mM DTT, followed by incubation at 100°C for 3 minutes. Each sample was applied to Any kD^{™} Mini-PROTEAN (registered trademark) TGX Stain-Free^{™} Protein Gel (Bio-Rad Laboratories, Inc.) and was then subjected to electrophoresis at a constant voltage of 200 V. The gel after electrophoresis was photographed using Chemi Doc MP Imaging system (Bio-Rad Laboratories, Inc.).

In Table 3, a sample in which a clear band was observed near the molecular weight of a lipase, compared to the empty vector, is indicated with "Good", a sample in which a slight band was observed is indicated with "Fair", and a sample in which no band was observed is indicated with "Poor". It was found that since a clear band of a lipase was observed in only 3 sequences among verified 16 sequences, lipases of the *Proteus*/*Yersinia* clade have a problem of low diversity of sequences that can be heterologously expressed in large quantities.

**[Table 3]**

| Name | Secretory expression |
|---|---|
| SspLip | Poor |
| PfLip | Good |
| EtLip | Fair |
| YeLip | Fair |
| AnLip | Poor |
| YfLip | Good |
| PspLip | Fair |
| PspLip2 | Poor |
| SaLip | Poor |
| BbLip | Poor |
| EspLip | Poor |
| AspLip | Poor |
| YeLip2 | Good |
| YmLip | Poor |
| EspLip2 | Fair |
| MiLip | Poor |

### (3) Evaluation of expression of lipase mutant

Lipase mutants were heterologously expressed by the same method as in (2). Lipase PS Amano SD (FUJIFILM Wako Pure Chemical Corporation) was dissolved in 20 mM Tris-HCl (pH 7.0), and the concentration was measured with DC Protein Assay (Bio-Rad Laboratories, Inc.) using BSA as a standard. The culture supernatant was subjected to SDS-PAGE in the same manner as in (2) together with Lipase PS Amano SD of known concentration. The lipase concentration of the culture supernatant was measured from the band strength using Lipase PS Amano SD as a standard (Table 4). In various lipases of the *Proteus*/*Yersinia* clade, the heterologous expression was drastically improved by site-directed modification.

**[Table 4]**

| Parent enzyme | Mutation | Productivity (mg/L) |
|---|---|---|
| SspLip (SEQ ID NO: 2) | Wild-type | 0 |
| | E1180 | 642 |
| PfLip (SEQ ID NO: 4) | Wild-type | 149 |
| | A89C | 191 |
| | V180L | 279 |
| | E31G | 234 |
| | F30Y E31G | 261 |
| | A89C V180L | 285 |
| | E31G A89C | 370 |
| | F30Y E31G A89C V180L | 919 |
| EtLip (SEQ ID NO: 6) | Wild-type | 12 |
| | I222A | 218 |
| | I222T | 243 |
| | I222H | 348 |
| | I222G | 355 |
| YeLip (SEQ ID NO: 8) | Wild-type | 55 |
| | V38K | 131 |
| | V124T E125P | 332 |
| | E125P | 299 |
| | S292G | 249 |
| | V38K S292G | 311 |
| | V124T E125P S292G | 543 |
| | V38K V124T E125P | 545 |
| | V38K E125P S292G | 373 |
| | V38K V124T E125P S292G | 563 |
| AnLip (SEQ ID NO: 10) | Wild-type | 0 |
| | C24Y | 278 |
| | R181G | 392 |
| | C24Y R181G | 506 |
| | C24Y F26Y A27G R181G | 750 |
| YfLip (SEQ ID NO: 12) | Wild-type | 312 |
| | G206Q | 781 |
| PspLip (SEQ ID NO: 14) | Wild-type | 26 |
| | F30Y D31G | 49 |
| | R76K | 170 |
| | F30Y D31G M179L | 133 |
| | R76K M179L | 293 |
| | F30Y D31G R76K M179L | 418 |
| PspLip2 (SEQ ID NO: 16) | Wild-type | 0 |
| | A89C | 14 |
| | V180L | 30 |
| | A89C V180L | 60 |

### (4) Calculation of sequence identity

Sequence identity between mutants with drastically improved heterologous expression (SspLip E118D, PfLip F30Y E31G A89C V180L, EtLip I222G, YeLip V38K V124T E125P S292G, AnLip C24Y F26Y A27G R181G, YfLip G206Q, PspLip F30Y D31G R76K M179L, PspLip2 A89C V180L) was calculated using Genetyx (Table 5). In lipases of the *Proteus*/*Yersinia* clade of these mutants, the diversity of sequences that can be heterologously expressed in large quantities was drastically increased.

**[Table 5]**

| No. | Mutant | 1 | 2 | 3 | 4 | 5 | 6 | 7 | 8 |
|---|---|---|---|---|---|---|---|---|---|
| 1 | SspLip E118D | | 60.7 | 69 | 66.2 | 53.5 | 70.1 | 57.9 | 60.9 |
| 2 | PfLip F30Y E31G A89C V180L | 60.7 | | 61 | 57.2 | 50 | 57.6 | 79 | 80.6 |
| 3 | EtLip I222G | 69 | 61 | | 67.6 | 55.6 | 67.3 | 59.3 | 61.2 |
| 4 | YeLip V38K V124T E125P S292G | 66.2 | 57.2 | 67.6 | | 53.1 | 64.2 | 57.2 | 57.8 |
| 5 | AnLip C24Y F26Y A27G R181G | 53.5 | 50 | 55.6 | 53.1 | | 55.9 | 52.8 | 49 |
| 6 | YfLip G206Q | 70.1 | 57.6 | 67.3 | 64.2 | 55.9 | | 56.9 | 58.5 |
| 7 | PspLip F30Y D31G R76K M179L | 57.9 | 79 | 59.3 | 57.2 | 52.8 | 56.9 | | 70.2 |
| 8 | PspLip2 A89C V180L | 60.9 | 80.6 | 61.2 | 57.8 | 49 | 58.5 | 70.2 | |

### (5) Evaluation of expression of PaLip mutant

PaLip (SEQ ID NO: 34), which is a lipase closely related to lipases of the *Proteus*/*Yersinia* clade, was heterologously expressed by the same method as in (2). In each well of a 96-well assay plate, 2 µL of a culture supernatant diluted by 100-fold with 20 mM Tris-HCl (pH 7.0) and 100 µL of a substrate solution were mixed, and the change in absorbance (OD/min) at 405 nm was measured at 30°C. As the substrate solution, 2 mM 4-nitrophenyl octanoate in 20 mM Tris-HCl (pH 7.0) was used. In wild-type PaLip, no esterase activity was detected in the culture supernatant, and it was thought that active-form lipase was not heterologously expressed. In contrast, in the PaLip M28Y mutant, esterase activity derived from a lipase was detected in the culture supernatant (Fig. 1).

## Claims

1. A lipase mutant selected from the following (a) to (v):
(a) a lipase mutant consisting of an amino acid sequence having an identity of at least 82% with the amino acid sequence of SEQ ID NO: 2 and having aspartic acid at a position corresponding to position 118 of the amino acid sequence of SEQ ID NO: 2;
(b) a lipase mutant consisting of an amino acid sequence having an identity of at least 80% with the amino acid sequence of SEQ ID NO: 4 and having tyrosine at a position corresponding to position 31 of the amino acid sequence of SEQ ID NO: 2;
(c) a lipase mutant consisting of an amino acid sequence having an identity of at least 91% with the amino acid sequence of SEQ ID NO: 4 and having glycine at a position corresponding to position 32 of the amino acid sequence of SEQ ID NO: 2;
(d) a lipase mutant consisting of an amino acid sequence having an identity of at least 80% with the amino acid sequence of SEQ ID NO: 4 and having cysteine at a position corresponding to position 90 of the amino acid sequence of SEQ ID NO: 2;
(e) a lipase mutant consisting of an amino acid sequence having an identity of at least 94% with the amino acid sequence of SEQ ID NO: 4 and having leucine at a position corresponding to position 181 of the amino acid sequence of SEQ ID NO: 2;
(f) a lipase mutant consisting of an amino acid sequence having an identity of at least 80% with the amino acid sequence of SEQ ID NO: 6 and having alanine, threonine, histidine, or glycine at a position corresponding to position 222 of the amino acid sequence of SEQ ID NO: 2;
(g) a lipase mutant consisting of an amino acid sequence having an identity of at least 80% with the amino acid sequence of SEQ ID NO: 8 and having lysine at a position corresponding to position 39 of the amino acid sequence of SEQ ID NO: 2;
(h) a lipase mutant consisting of an amino acid sequence having an identity of at least 80% with the amino acid sequence of SEQ ID NO: 8 and having threonine at a position corresponding to position 125 of the amino acid sequence of SEQ ID NO: 2;
(i) a lipase mutant consisting of an amino acid sequence having an identity of at least 80% with the amino acid sequence of SEQ ID NO: 8 and having proline at a position corresponding to position 126 of the amino acid sequence of SEQ ID NO: 2;
(j) a lipase mutant consisting of an amino acid sequence having an identity of at least 80% with the amino acid sequence of SEQ ID NO: 8 and having glycine at a position corresponding to position 293 of the amino acid sequence of SEQ ID NO: 2;
(k) a lipase mutant consisting of an amino acid sequence having an identity of at least 80% with the amino acid sequence of SEQ ID NO: 10 and having tyrosine at a position corresponding to position 29 of the amino acid sequence of SEQ ID NO: 2;
(l) a lipase mutant consisting of an amino acid sequence having an identity of at least 80% with the amino acid sequence of SEQ ID NO: 10 and having tyrosine at a position corresponding to position 31 of the amino acid sequence of SEQ ID NO: 2;
(m) a lipase mutant consisting of an amino acid sequence having an identity of at least 80% with the amino acid sequence of SEQ ID NO: 10 and having glycine at a position corresponding to position 32 of the amino acid sequence of SEQ ID NO: 2;
(n) a lipase mutant consisting of an amino acid sequence having an identity of at least 80% with the amino acid sequence of SEQ ID NO: 10 and having glycine at a position corresponding to position 186 of the amino acid sequence of SEQ ID NO: 2;
(o) a lipase mutant consisting of an amino acid sequence having an identity of at least 80% with the amino acid sequence of SEQ ID NO: 12 and having glutamine at a position corresponding to position 206 of the amino acid sequence of SEQ ID NO: 2;
(p) a lipase mutant consisting of an amino acid sequence having an identity of at least 80% with the amino acid sequence of SEQ ID NO: 14 and having tyrosine at a position corresponding to position 31 of the amino acid sequence of SEQ ID NO: 2;
(q) a lipase mutant consisting of an amino acid sequence having an identity of at least 80% with the amino acid sequence of SEQ ID NO: 14 and having glycine at a position corresponding to position 32 of the amino acid sequence of SEQ ID NO: 2;
(r) a lipase mutant consisting of an amino acid sequence having an identity of at least 97% with the amino acid sequence of SEQ ID NO: 14 and having lysine at a position corresponding to position 77 of the amino acid sequence of SEQ ID NO: 2;
(s) a lipase mutant consisting of an amino acid sequence having an identity of at least 83% with the amino acid sequence of SEQ ID NO: 14 and having leucine at a position corresponding to position 181 of the amino acid sequence of SEQ ID NO: 2;
(t) a lipase mutant consisting of an amino acid sequence having an identity of at least 80% with the amino acid sequence of SEQ ID NO: 16 and having cysteine at a position corresponding to position 90 of the amino acid sequence of SEQ ID NO: 2;
(u) a lipase mutant consisting of an amino acid sequence having an identity of at least 81% with the amino acid sequence of SEQ ID NO: 16 and having leucine at a position corresponding to position 181 of the amino acid sequence of SEQ ID NO: 2; and
(v) a lipase mutant consisting of an amino acid sequence having an identity of at least 80% with the amino acid sequence of SEQ ID NO: 34 and having tyrosine at a position corresponding to position 29 of the amino acid sequence of SEQ ID NO: 2.

2. A lipase mutant selected from the following (aa) to (ae):
(aa) a lipase mutant consisting of an amino acid sequence having an identity of at least 80% with the amino acid sequence of SEQ ID NO: 4 and having at least two amino acid residues selected from tyrosine at a position corresponding to position 31, glycine at a position corresponding to position 32, cysteine at a position corresponding to position 90, and leucine at a position corresponding to position 181 of the amino acid sequence of SEQ ID NO: 2;
(ab) a lipase mutant consisting of an amino acid sequence having an identity of at least 80% with the amino acid sequence of SEQ ID NO: 8 and having at least two amino acid residues selected from lysine at a position corresponding to position 39, threonine at a position corresponding to position 125, proline at a position corresponding to position 126, and glycine at a position corresponding to position 293 of the amino acid sequence of SEQ ID NO: 2;
(ac) a lipase mutant consisting of an amino acid sequence having an identity of at least 80% with the amino acid sequence of SEQ ID NO: 10 and having at least two amino acid residues selected from tyrosine at a position corresponding to position 29, tyrosine at a position corresponding to position 31, glycine at a position corresponding to position 32, and glycine at a position corresponding to position 186 of the amino acid sequence of SEQ ID NO: 2;
(ad) a lipase mutant consisting of an amino acid sequence having an identity of at least 83% with the amino acid sequence of SEQ ID NO: 14 and having at least two amino acid residues selected from tyrosine at a position corresponding to position 31, glycine at a position corresponding to position 32, lysine at a position corresponding to position 77, and leucine at a position corresponding to position 181 of the amino acid sequence of SEQ ID NO: 2; and
(ae) a lipase mutant consisting of an amino acid sequence having an identity of at least 80% with the amino acid sequence of SEQ ID NO: 16 and having cysteine at a position corresponding to position 90 and leucine at a position corresponding to position 181 of the amino acid sequence of SEQ ID NO: 2.

3. The lipase mutant according to claim 1, wherein the lipase mutant is a lipase mutant selected from the (a), (c) to (g), (i) to (k), (n), (o), (r), and (t) to (v).

4. The lipase mutant according to claim 2, wherein the lipase mutant is a lipase mutant selected from the following (aa-1) to (ae):
(aa-1) a lipase mutant that consists of an amino acid sequence having an identity of at least 80% with the amino acid sequence of SEQ ID NO: 4 and has tyrosine at a position corresponding to position 31 and glycine at a position corresponding to position 32 of the amino acid sequence of SEQ ID NO: 2;
(aa-2) a lipase mutant that consists of an amino acid sequence having an identity of at least 80% with the amino acid sequence of SEQ ID NO: 4 and has cysteine at a position corresponding to position 90 and leucine at a position corresponding to position 181 of the amino acid sequence of SEQ ID NO: 2;
(aa-3) a lipase mutant that consists of an amino acid sequence having an identity of at least 80% with the amino acid sequence of SEQ ID NO: 4 and has glycine at a position corresponding to position 32 and cysteine at a position corresponding to position 90 of the amino acid sequence of SEQ ID NO: 2;
(aa-4) a lipase mutant that consists of an amino acid sequence having an identity of at least 80% with the amino acid sequence of SEQ ID NO: 4 and has tyrosine at a position corresponding to position 31, glycine at a position corresponding to position 32, cysteine at a position corresponding to position 90, and L at a position corresponding to position 181 of the amino acid sequence of SEQ ID NO: 2;
(ab-1) a lipase mutant that consists of an amino acid sequence having an identity of at least 80% with the amino acid sequence of SEQ ID NO: 8 and has threonine at a position corresponding to position 125 and proline at a position corresponding to position 126 of the amino acid sequence of SEQ ID NO: 2;
(ab-2) a lipase mutant that consists of an amino acid sequence having an identity of at least 80% with the amino acid sequence of SEQ ID NO: 8 and has lysine at a position corresponding to position 39 and glycine at a position corresponding to position 293 of the amino acid sequence of SEQ ID NO: 2;
(ab-3) a lipase mutant that consists of an amino acid sequence having an identity of at least 80% with the amino acid sequence of SEQ ID NO: 8 and has threonine at a position corresponding to position 125, proline at a position corresponding to position 126, and glycine at a position corresponding to position 293 of the amino acid sequence of SEQ ID NO: 2;
(ab-4) a lipase mutant that consists of an amino acid sequence having an identity of at least 80% with the amino acid sequence of SEQ ID NO: 8 and has lysine at a position corresponding to position 39, threonine at a position corresponding to position 125, and proline at a position corresponding to position 126 of the amino acid sequence of SEQ ID NO: 2;
(ab-5) a lipase mutant that consists of an amino acid sequence having an identity of at least 80% with the amino acid sequence of SEQ ID NO: 8 and has lysine at a position corresponding to position 39, proline at a position corresponding to position 126, and glycine at a position corresponding to position 293 of the amino acid sequence of SEQ ID NO: 2;
(ab-6) a lipase mutant that consists of an amino acid sequence having an identity of at least 80% with the amino acid sequence of SEQ ID NO: 8 and has lysine at a position corresponding to position 39, threonine at a position corresponding to position 125, proline at a position corresponding to position 126, and glycine at a position corresponding to position 293 of the amino acid sequence of SEQ ID NO: 2;
(ac-1) a lipase mutant that consists of an amino acid sequence having an identity of at least 80% with the amino acid sequence of SEQ ID NO: 10 and has tyrosine at a position corresponding to position 29 and glycine at a position corresponding to position 186 of the amino acid sequence of SEQ ID NO: 2;
(ac-2) a lipase mutant that consists of an amino acid sequence having an identity of at least 80% with the amino acid sequence of SEQ ID NO: 10 and has tyrosine at a position corresponding to position 29, tyrosine at a position corresponding to position 31, glycine at a position corresponding to position 32, and glycine at a position corresponding to position 186 of the amino acid sequence of SEQ ID NO: 2;
(ad-1) a lipase mutant that consists of an amino acid sequence having an identity of at least 83% with the amino acid sequence of SEQ ID NO: 14 and has tyrosine at a position corresponding to position 31 and glycine at a position corresponding to position 32 of the amino acid sequence of SEQ ID NO: 2;
(ad-2) a lipase mutant that consists of an amino acid sequence having an identity of at least 83% with the amino acid sequence of SEQ ID NO: 14 and has lysine at a position corresponding to position 77 and leucine at a position corresponding to position 181 of the amino acid sequence of SEQ ID NO: 2;
(ad-3) a lipase mutant that consists of an amino acid sequence having an identity of at least 83% with the amino acid sequence of SEQ ID NO: 14 and has tyrosine at a position corresponding to position 31, glycine at a position corresponding to position 32, and leucine at a position corresponding to position 181 of the amino acid sequence of SEQ ID NO: 2;
(ad-4) a lipase mutant that consists of an amino acid sequence having an identity of at least 83% with the amino acid sequence of SEQ ID NO: 14 and has tyrosine at a position corresponding to position 31, glycine at a position corresponding to position 32, lysine at a position corresponding to position 77, and leucine at a position corresponding to position 181 of the amino acid sequence of SEQ ID NO: 2; and
(ae) a lipase mutant that consists of an amino acid sequence having an identity of at least 80% with the amino acid sequence of SEQ ID NO: 16 and has cysteine at a position corresponding to position 90 and leucine at a position corresponding to position 181 of the amino acid sequence of SEQ ID NO: 2.

5. An enzyme composition comprising the lipase mutant according to any one of claims 1 to 4.

6. A polynucleotide encoding the lipase mutant according to any one of claims 1 to 4.

7. A vector or DNA fragment comprising the polynucleotide according to claim 6.

8. A transformed cell comprising the vector or DNA fragment according to claim 7.

9. The transformed cell according to claim 8, wherein the transformed cell is a microorganism.

10. The transformed cell according to claim 9, wherein the transformed cell is *Escherichia coli* or a *Bacillus* bacterium.

11. A method for producing a lipase mutant, comprising a step of culturing the transformed cell according to any one of claims 8 to 10.

12. A method for manufacturing a lipase mutant, comprising a step selected from the following (i) to (xxii) :
(i) a step of substituting an amino acid residue at a position corresponding to position 118 of the amino acid sequence of SEQ ID NO: 2 with aspartic acid in a polypeptide that consists of an amino acid sequence having an identity of at least 82% with the amino acid sequence of SEQ ID NO: 2 and has lipase activity;
(ii) a step of substituting an amino acid residue at a position corresponding to position 31 of the amino acid sequence of SEQ ID NO: 2 with tyrosine in a polypeptide that consists of an amino acid sequence having an identity of at least 80% with the amino acid sequence of SEQ ID NO: 4 and has lipase activity;
(iii) a step of substituting an amino acid residue at a position corresponding to position 32 of the amino acid sequence of SEQ ID NO: 2 with glycine in a polypeptide that consists of an amino acid sequence having an identity of at least 91% with the amino acid sequence of SEQ ID NO: 4 and has lipase activity;
(iv) a step of substituting an amino acid residue at a position corresponding to position 90 of the amino acid sequence of SEQ ID NO: 2 with cysteine in a polypeptide that consists of an amino acid sequence having an identity of at least 80% with the amino acid sequence of SEQ ID NO: 4 and has lipase activity;
(v) a step of substituting an amino acid residue at a position corresponding to position 181 of the amino acid sequence of SEQ ID NO: 2 with leucine in a polypeptide that consists of an amino acid sequence having an identity of at least 94% with the amino acid sequence of SEQ ID NO: 4 and has lipase activity;
(vi) a step of substituting an amino acid residue at a position corresponding to position 222 of the amino acid sequence of SEQ ID NO: 2 with alanine, threonine, histidine, or glycine in a polypeptide that consists of an amino acid sequence having an identity of at least 80% with the amino acid sequence of SEQ ID NO: 6 and has lipase activity;
(vii) a step of substituting an amino acid residue at a position corresponding to position 39 of the amino acid sequence of SEQ ID NO: 2 with lysine in a polypeptide that consists of an amino acid sequence having an identity of at least 80% with the amino acid sequence of SEQ ID NO: 8 and has lipase activity;
(viii) a step of substituting an amino acid residue at a position corresponding to position 125 of the amino acid sequence of SEQ ID NO: 2 with threonine in a polypeptide that consists of an amino acid sequence having an identity of at least 80% with the amino acid sequence of SEQ ID NO: 8 and has lipase activity;
(ix) a step of substituting an amino acid residue at a position corresponding to position 126 of the amino acid sequence of SEQ ID NO: 2 with proline in a polypeptide that consists of an amino acid sequence having an identity of at least 80% with the amino acid sequence of SEQ ID NO: 8 and has lipase activity;
(x) a step of substituting an amino acid residue at a position corresponding to position 293 of the amino acid sequence of SEQ ID NO: 2 with glycine in a polypeptide that consists of an amino acid sequence having an identity of at least 80% with the amino acid sequence of SEQ ID NO: 8 and has lipase activity;
(xi) a step of substituting an amino acid residue at a position corresponding to position 29 of the amino acid sequence of SEQ ID NO: 2 with tyrosine in a polypeptide that consists of an amino acid sequence having an identity of at least 80% with the amino acid sequence of SEQ ID NO: 10 and has lipase activity;
(xii) a step of substituting an amino acid residue at a position corresponding to position 31 of the amino acid sequence of SEQ ID NO: 2 with tyrosine in a polypeptide that consists of an amino acid sequence having an identity of at least 80% with the amino acid sequence of SEQ ID NO: 10 and has lipase activity;
(xiii) a step of substituting an amino acid residue at a position corresponding to position 32 of the amino acid sequence of SEQ ID NO: 2 with glycine in a polypeptide that consists of an amino acid sequence having an identity of at least 80% with the amino acid sequence of SEQ ID NO: 10 and has lipase activity;
(xiv) a step of substituting an amino acid residue at a position corresponding to position 186 of the amino acid sequence of SEQ ID NO: 2 with glycine in a polypeptide that consists of an amino acid sequence having an identity of at least 80% with the amino acid sequence of SEQ ID NO: 10 and has lipase activity;
(xv) a step of substituting an amino acid residue at a position corresponding to position 206 of the amino acid sequence of SEQ ID NO: 2 with glutamine in a polypeptide that consists of an amino acid sequence having an identity of at least 80% with the amino acid sequence of SEQ ID NO: 12 and has lipase activity;
(xvi) a step of substituting an amino acid residue at a position corresponding to position 31 of the amino acid sequence of SEQ ID NO: 2 with tyrosine in a polypeptide that consists of an amino acid sequence having an identity of at least 80% with the amino acid sequence of SEQ ID NO: 14 and has lipase activity;
(xvii) a step of substituting an amino acid residue at a position corresponding to position 32 of the amino acid sequence of SEQ ID NO: 2 with glycine in a polypeptide that consists of an amino acid sequence having an identity of at least 80% with the amino acid sequence of SEQ ID NO: 14 and has lipase activity;
(xviii) a step of substituting an amino acid residue at a position corresponding to position 77 of the amino acid sequence of SEQ ID NO: 2 with lysine in a polypeptide that consists of an amino acid sequence having an identity of at least 97% with the amino acid sequence of SEQ ID NO: 14 and has lipase activity;
(xix) a step of substituting an amino acid residue at a position corresponding to position 181 of the amino acid sequence of SEQ ID NO: 2 with leucine in a polypeptide that consists of an amino acid sequence having an identity of at least 83% with the amino acid sequence of SEQ ID NO: 14 and has lipase activity;
(xx) a step of substituting an amino acid residue at a position corresponding to position 90 of the amino acid sequence of SEQ ID NO: 2 with cysteine in a polypeptide that consists of an amino acid sequence having an identity of at least 80% with the amino acid sequence of SEQ ID NO: 16 and has lipase activity;
(xxi) a step of substituting an amino acid residue at a position corresponding to position 181 of the amino acid sequence of SEQ ID NO: 2 with leucine in a polypeptide that consists of an amino acid sequence having an identity of at least 81% with the amino acid sequence of SEQ ID NO: 16 and has lipase activity; and
(xxii) a step of substituting an amino acid residue at a position corresponding to position 29 of the amino acid sequence of SEQ ID NO: 2 with tyrosine in a polypeptide that consists of an amino acid sequence having an identity of at least 80% with the amino acid sequence of SEQ ID NO: 34 and has lipase activity.

13. A method for producing a lipase mutant, comprising a step selected from the following (xxiii) to (xxvii) :
(xxiii) a step of substituting at least two amino acid residues at positions selected from positions corresponding to positions 31, 32, 90, and 181 of the amino acid sequence of SEQ ID NO: 2 with tyrosine at a position corresponding to position 31, glycine at a position corresponding to position 32, cysteine at a position corresponding to position 90, and leucine at a position corresponding to position 181 in a polypeptide that consists of an amino acid sequence having an identity of at least 80% with the amino acid sequence of SEQ ID NO: 4 and has lipase activity;
(xxiv) a step of substituting at least two amino acid residues at positions selected from positions corresponding to positions 39, 125, 126, and 293 of the amino acid sequence of SEQ ID NO: 2 with lysine at a position corresponding to position 39, threonine at a position corresponding to position 125, proline at a position corresponding to position 126, and glycine at a position corresponding to position 293 in a polypeptide that consists of an amino acid sequence having an identity of at least 80% with the amino acid sequence of SEQ ID NO: 8 and has lipase activity;
(xxv) a step of substituting at least two amino acid residues at positions selected from positions corresponding to positions 29, 31, 32, and 186 of the amino acid sequence of SEQ ID NO: 2 with tyrosine at a position corresponding to position 29, tyrosine at a position corresponding to position 31, glycine at a position corresponding to position 32, and glycine at a position corresponding to position 186 in a polypeptide that consists of an amino acid sequence having an identity of at least 80% with the amino acid sequence of SEQ ID NO: 10 and has lipase activity;
(xxvi) a step of substituting at least two amino acid residues at positions selected from positions corresponding to positions 31, 32, 77, and 181 of the amino acid sequence of SEQ ID NO: 2 with tyrosine at a position corresponding to position 31, glycine at a position corresponding to position 32, lysine at a position corresponding to position 77, and leucine at a position corresponding to position 181 in a polypeptide that consists of an amino acid sequence having an identity of at least 83% with the amino acid sequence of SEQ ID NO: 14 and has lipase activity; and
(xxvii) a step of substituting an amino acid residue at a position corresponding to position 90 of the amino acid sequence of SEQ ID NO: 2 with cysteine and an amino acid residue at a position corresponding to position 181 with leucine in a polypeptide that consists of an amino acid sequence having an identity of at least 80% with the amino acid sequence of SEQ ID NO: 16 and has lipase activity.

14. A method for improving heterologous expression of a lipase, comprising a step selected from the following (i) to (xxii):
(i) a step of substituting an amino acid residue at a position corresponding to position 118 of the amino acid sequence of SEQ ID NO: 2 with aspartic acid in a polypeptide that consists of an amino acid sequence having an identity of at least 82% with the amino acid sequence of SEQ ID NO: 2 and has lipase activity;
(ii) a step of substituting an amino acid residue at a position corresponding to position 31 of the amino acid sequence of SEQ ID NO: 2 with tyrosine in a polypeptide that consists of an amino acid sequence having an identity of at least 80% with the amino acid sequence of SEQ ID NO: 4 and has lipase activity;
(iii) a step of substituting an amino acid residue at a position corresponding to position 32 of the amino acid sequence of SEQ ID NO: 2 with glycine in a polypeptide that consists of an amino acid sequence having an identity of at least 91% with the amino acid sequence of SEQ ID NO: 4 and has lipase activity;
(iv) a step of substituting an amino acid residue at a position corresponding to position 90 of the amino acid sequence of SEQ ID NO: 2 with cysteine in a polypeptide that consists of an amino acid sequence having an identity of at least 80% with the amino acid sequence of SEQ ID NO: 4 and has lipase activity;
(v) a step of substituting an amino acid residue at a position corresponding to position 181 of the amino acid sequence of SEQ ID NO: 2 with leucine in a polypeptide that consists of an amino acid sequence having an identity of at least 94% with the amino acid sequence of SEQ ID NO: 4 and has lipase activity;
(vi) a step of substituting an amino acid residue at a position corresponding to position 222 of the amino acid sequence of SEQ ID NO: 2 with alanine, threonine, histidine, or glycine in a polypeptide that consists of an amino acid sequence having an identity of at least 80% with the amino acid sequence of SEQ ID NO: 6 and has lipase activity;
(vii) a step of substituting an amino acid residue at a position corresponding to position 39 of the amino acid sequence of SEQ ID NO: 2 with lysine in a polypeptide that consists of an amino acid sequence having an identity of at least 80% with the amino acid sequence of SEQ ID NO: 8 and has lipase activity;
(viii) a step of substituting an amino acid residue at a position corresponding to position 125 of the amino acid sequence of SEQ ID NO: 2 with threonine in a polypeptide that consists of an amino acid sequence having an identity of at least 80% with the amino acid sequence of SEQ ID NO: 8 and has lipase activity;
(ix) a step of substituting an amino acid residue at a position corresponding to position 126 of the amino acid sequence of SEQ ID NO: 2 with proline in a polypeptide that consists of an amino acid sequence having an identity of at least 80% with the amino acid sequence of SEQ ID NO: 8 and has lipase activity;
(x) a step of substituting an amino acid residue at a position corresponding to position 293 of the amino acid sequence of SEQ ID NO: 2 with glycine in a polypeptide that consists of an amino acid sequence having an identity of at least 80% with the amino acid sequence of SEQ ID NO: 8 and has lipase activity;
(xi) a step of substituting an amino acid residue in a polypeptide that consists of an amino acid sequence having an identity of at least 80% with the amino acid sequence of SEQ ID NO: 10 and has lipase activity with tyrosine at a position corresponding to position 29 of the amino acid sequence of SEQ ID NO: 2;
(xii) a step of substituting an amino acid residue at a position corresponding to position 31 of the amino acid sequence of SEQ ID NO: 2 with tyrosine in a polypeptide that consists of an amino acid sequence having an identity of at least 80% with the amino acid sequence of SEQ ID NO: 10 and has lipase activity;
(xiii) a step of substituting an amino acid residue at a position corresponding to position 32 of the amino acid sequence of SEQ ID NO: 2 with glycine in a polypeptide that consists of an amino acid sequence having an identity of at least 80% with the amino acid sequence of SEQ ID NO: 10 and has lipase activity;
(xiv) a step of substituting an amino acid residue at a position corresponding to position 186 of the amino acid sequence of SEQ ID NO: 2 with glycine in a polypeptide that consists of an amino acid sequence having an identity of at least 80% with the amino acid sequence of SEQ ID NO: 10 and has lipase activity;
(xv) a step of substituting an amino acid residue at a position corresponding to position 206 of the amino acid sequence of SEQ ID NO: 2 with glutamine in a polypeptide that consists of an amino acid sequence having an identity of at least 80% with the amino acid sequence of SEQ ID NO: 12 and has lipase activity;
(xvi) a step of substituting an amino acid residue at a position corresponding to position 31 of the amino acid sequence of SEQ ID NO: 2 with tyrosine in a polypeptide that consists of an amino acid sequence having an identity of at least 80% with the amino acid sequence of SEQ ID NO: 14 and has lipase activity;
(xvii) a step of substituting an amino acid residue at a position corresponding to position 32 of the amino acid sequence of SEQ ID NO: 2 with glycine in a polypeptide that consists of an amino acid sequence having an identity of at least 80% with the amino acid sequence of SEQ ID NO: 14 and has lipase activity;
(xviii) a step of substituting an amino acid residue at a position corresponding to position 77 of the amino acid sequence of SEQ ID NO: 2 with lysine in a polypeptide that consists of an amino acid sequence having an identity of at least 97% with the amino acid sequence of SEQ ID NO: 14 and has lipase activity;
(xix) a step of substituting an amino acid residue at a position corresponding to position 181 of the amino acid sequence of SEQ ID NO: 2 with leucine in a polypeptide that consists of an amino acid sequence having an identity of at least 83% with the amino acid sequence of SEQ ID NO: 14 and has lipase activity;
(xx) a step of substituting an amino acid residue at a position corresponding to position 90 of the amino acid sequence of SEQ ID NO: 2 with cysteine in a polypeptide that consists of an amino acid sequence having an identity of at least 80% with the amino acid sequence of SEQ ID NO: 16 and has lipase activity;
(xxi) a step of substituting an amino acid residue at a position corresponding to position 181 of the amino acid sequence of SEQ ID NO: 2 with leucine in a polypeptide that consists of an amino acid sequence having an identity of at least 81% with the amino acid sequence of SEQ ID NO: 16 and has lipase activity; and
(xxii) a step of substituting an amino acid residue at a position corresponding to position 29 of the amino acid sequence of SEQ ID NO: 2 with tyrosine in a polypeptide that consists of an amino acid sequence having an identity of at least 80% with the amino acid sequence of SEQ ID NO: 34 and has lipase activity.

15. A method for improving heterologous expression of a lipase, comprising a step selected from the following (xxiii) to (xxvii):
(xxiii) a step of substituting at least two amino acid residues at positions selected from positions corresponding to positions 31, 32, 90, and 181 of the amino acid sequence of SEQ ID NO: 2 with tyrosine at a position corresponding to position 31, glycine at a position corresponding to position 32, cysteine at a position corresponding to position 90, and leucine at a position corresponding to position 181 in a polypeptide that consists of an amino acid sequence having an identity of at least 80% with the amino acid sequence of SEQ ID NO: 4 and has lipase activity;
(xxiv) a step of substituting at least two amino acid residues at positions selected from positions corresponding to positions 39, 125, 126, and 293 of the amino acid sequence of SEQ ID NO: 2 with lysine at a position corresponding to position 39, threonine at a position corresponding to position 125, proline at a position corresponding to position 126, and glycine at a position corresponding to position 293 in a polypeptide that consists of an amino acid sequence having an identity of at least 80% with the amino acid sequence of SEQ ID NO: 8 and has lipase activity;
(xxv) a step of substituting at least two amino acid residues at positions selected from positions corresponding to positions 29, 31, 32, and 186 of the amino acid sequence of SEQ ID NO: 2 with tyrosine at a position corresponding to position 29, tyrosine at a position corresponding to position 31, glycine at a position corresponding to position 32, and glycine at a position corresponding to position 186 in a polypeptide that consists of an amino acid sequence having an identity of at least 80% with the amino acid sequence of SEQ ID NO: 10 and has lipase activity;
(xxvi) a step of substituting at least two amino acid residues at positions selected from positions corresponding to positions 31, 32, 77, and 181 of the amino acid sequence of SEQ ID NO: 2 with tyrosine at a position corresponding to position 31, glycine at a position corresponding to position 32, lysine at a position corresponding to position 77, and leucine at a position corresponding to position 181 in a polypeptide that consists of an amino acid sequence having an identity of at least 83% with the amino acid sequence of SEQ ID NO: 14 and has lipase activity; and
(xxvii) a step of substituting an amino acid residue at a position corresponding to position 90 of the amino acid sequence of SEQ ID NO: 2 with cysteine and an amino acid residue at a position corresponding to position 181 with leucine in a polypeptide that consists of an amino acid sequence having an identity of at least 80% with the amino acid sequence of SEQ ID NO: 16 and has lipase activity.
